# EUROPÄISCHE PATENTANMELDUNG

(11) **EP 4 541 347 A1**
(43) Veröffentlichungstag der Anmeldung: **23.04.2025**
(21) Anmeldenummer: 24183813.5
(22) Anmeldetag: 21.06.2024
(51) Int. Cl.: A61K 9/00, A61K 9/107, A61K 47/14, A61K 47/44, A61K 31/00, A61P 25/00, A61P 25/02, A61P 25/04

(54) **FLÜSSIGE PHARMAZEUTISCHE REZEPTUR ZUR INTRAVENÖSEN, SUBKUTANEN, ODER INTRAMUSKULÄREN APPLIKATION EINER ODER MEHRERER CANNABINOIDE**

(30) Priorität: 20.10.2023 DE 102023128848
(71) Anmelder: Sativari UG (haftungsbeschränkt), 63329 Egelsbach (DE)
(72) Erfinder: Krüger, Volker, 34329 Nieste (DE)
(74) Vertreter: Ziebig Hengelhaupt Intellectual Property Attorneys Patentanwaltskanzlei PartGmbB

(57) **Zusammenfassung**

Die vorliegende Erfindung betrifft eine Emulsion bestehend aus mindestens einer Ölphase und mindestens einer wässrigen Phase, dadurch gekennzeichnet, dass die Emulsion in der mindestens einen Ölphase mindestens ein Cannabinoid enthält. Ferner wird die Emulsion zur Verwendung als Arzneimittel verwendet.

## Beschreibung

### TECHNISCHES GEBIET DER ERFINDUNG

Die Erfindung betrifft cannabinoid-haltige pharmazeutische Zubereitungen zur parenteralen Applikation. Im Speziellen umfassen die pharmazeutischen Zubereitungen Emulsionen.

Als Wirkstoff sind alle Substanzen der 12 Cannabinoid-Gruppen (siehe Pkt A, Cannabinoide als Bestandteil der Cannabinoid-Emulsion) von Cannabis sativa bzw. Cannabis indica sowie deren synthetisch hergestellte Wirkstoff-Analogons möglich. Im Speziellen sind die Substanzen, die unter Punkt B. stehen, zu berücksichtigen.

### Cannabinoide als Bestandteil der Cannabinoid-Emulsion

A. Folgende Cannabinoid-Typen können als Wirkstoff eingesetzt werden:
   1. Tetrahydrocannabinol-artige
   2. Cannabidiol-artige
   3. Cannabigerol-artige
   4. Cannabichromen-artige
   5. Cannabicyclol-artige
   6. Cannabielsoin-artige
   7. Cannabitriol-artige
   8. Cannabinol-artige
   9. Cannabinodiol-artige
   10. Cannabicitran-artige
   11. Cannabichromanon-artige
   12. Isocannabinoide
B. Im Speziellen:
   1. Δ9-Tetrahydrocannabinol (THC/Δ9-THC)
   2. Δ8-Tetrahydrocannabinol (Δ8-THC)
   3. Δ9-Tetrahydrocannabivarin (Δ9-THCV)
   4. Cannabidiol (CBD)
   5. Cannabinol (CBN)
   6. Cannabigerol (CBG)
   7. Cannabidivarin (CBDV)
   8. Cannabichromen (CBC)
   9. Cannabicyclol (CBL)
   10. Cannabielsoin (CBE)
   11. Cannabitriol (CBT)
   12. Cannabinodiol (CBND)
   13. Cannabicitran (CBT)
   14. Cannabichromanon (CBCN)
   15. Tetrahydrocannabinolsäure (THCA)
   16. Tetrahydrocannabivarin (THCV)

### HINTERGRUND DER ERFINDUNG

Der menschliche Organismus produziert Endocannabinoide, die an den Cannabinoid-Rezeptoren binden. Somit stellt der pharmazeutische Einsatz von exogenen Cannabinoiden eine Therapie mit körperähnlichen Substanzen dar, was sowohl die therapeutische Wirksamkeit als auch das Nebenwirkungsprofil der Cannabinoide erklären könnte.

Das menschliche Gehirn und andere Organe enthalten natürlich vorkommende Cannabinoid-Rezeptoren (CB) und die chemischen Substanzen (Cannabinoide), die sich an sie binden. Dies wird als menschliches Endocannabinoidsystem (ECS) bezeichnet. Die Aufgabe des Endocannabinoidsystem besteht darin, die normale Funktionsfähigkeit unseres Körpers aufrechtzuerhalten, indem es die Funktion anderer Systeme beeinflusst. Es spielt eine entscheidende Rolle in unserem Nervensystem und reguliert mehrere physiologische Prozesse. Dazu gehört die Anpassung unserer Reaktion auf Schmerzen, Appetit, Verdauung, Schlaf, Stimmung, Entzündungen und Gedächtnis. Das ECS beeinflusst auch die Anfallsschwelle (z. B. bei Epilepsie), die Koordination und andere Prozesse wie das Immunsystem, die Herzfunktion und die sensorische Integration (Berührung, Gleichgewicht, Raumgefühl), Fruchtbarkeit, Knochenphysiologie, das zentrale Stressreaktionssystem (HPAA), neuronale Entwicklung und den Augendruck.

Die Literatur beschreibt zwei Rezeptortypen, welche beiden in unterschiedlicher Anzahl im zentralen Nervensystem und der Peripherie des Organismus zu finden sind: Der Cannabinoid-Rezeptor 1 (CB1) kommt vorwiegend in den Nervenzellen, hauptsächlich in verschiedenen Teilen des Gehirns (Hippocampus, Basalganglien und Kleinhirn), vor. Cannabinoide, die an diese Rezeptoren binden, bilden eine wichtige Schutzfunktion, indem sie die Über- und Unteraktivität, ausgelöst durch Neurotransmitter wie Dopamin, Serotonin, Noradrenalin und Glutamat, ausbalancieren. Aus diesem Grund sind psychoaktive Cannabinoide (z.B. Δ9-Tetrahydrocannabinol) in der Lage Symptomen wie Schmerzen, Übelkeit, epileptischen Anfällen, Muskelspasmen durch Multiple Sklerose und Angststörungen entgegenzuwirken. CB1-Rezeptoren, die sich im peripheren Nervensystem befinden, zeigen entsprechende Effekte im Magen-Darm-Trakt.

Die zweite Art Rezeptor, der Cannabinoid-Rezeptor 2 (CB2), befindet sich vorzugsweise auf Zellen des Immunsystems (T-Zellen, B-Zellen, neutrophile Granulozyten, Monozyten und NK-Zellen), den Osteoblasten (Knochenaufbau) und Osteoklasten (Knochenabbau).

Der CB1-Rezeptor kommt hauptsächlich im Gehirn und im Zentralnervensystem vor. CB1 kommt jedoch auch in bestimmten Geweben und Organen vor, beispielsweise in der Lunge, der Leber und den Nieren.

Beispielsweise befindet sich der CB1-Rezeptor in einer Reihe von Regionen des Gehirns, die verschiedene Körper- und Verhaltensfunktionen steuern. Dadurch beeinflussen Cannabinoide sensorische und motorische Reaktionsfähigkeit (Bewegung), Herzfrequenz, emotionale Reaktionen, Appetit und Übelkeit/Erbrechen, Schmerzempfindlichkeit, Lernen und Gedächtnis sowie Entscheidungsfindung auf hoher Ebene.

Die CB2-Rezeptoren kommen vor allem auf bestimmten Zellen des Immunsystems, im Magen-Darm-Trakt und in immunbezogenen Organen wie Milz und Mandeln vor.

Gruppen von Cannabinoiden:
- Endocannabinoide
- Phytocannabinoide
- Synthetische Cannabinoide (z.B. Dronabinol, Nabilone)

Von den 12 Cannabinoid-Typen (siehe Cannabinoide als Bestandteil der Cannabinoid-Emulsion) sind Δ9-Tetrahydrocannabinol und Cannabidiol momentan noch am besten untersucht. Jedoch binden auch die restlichen Cannabinoide unterschiedlich stark an die CB1- und CB2-Rezeptoren, sodass sich zukünftig eine Vielzahl an pharmakologischen Anwendungen ergeben werden. Zum momentanen Zeitpunkt stehen gerade Applikationen als Analgetikum, Spasmolytikum, Antiemetikum, Sedativum, Antiphlogistikum und Antirheumatikum im Fokus, jedoch können sich die positiven Effekte der Cannabinoide gerade in den Bereichen Immunologie und Alzheimer Erkrankung als besonders interessant herausstellen.

Von den über 100 inzwischen bekannten Cannabinoiden sind derzeit vor allem die Cannabinoide Δ9-Tetrahydrocannabinol (THC) und Cannabidiol (CBD) als medizinische Wirkstoffe bekannt. Unter anderem ist das halbsynthetische THC, Dronabinol, in Deutschland und anderen Staaten als verschreibungspflichtiges Betäubungsmittel (Handelsname Marinol) bei Anorexie und Kachexie bei HIV- und AIDS-Patienten sowie als Antiemetikum bei Übelkeit und Erbrechen unter Zytostatika- bzw. Bestrahlungstherapie im Rahmen einer Krebstherapie anwendbar. Das vollsynthetische THC-Derivat Nabilon hat eine ähnliche Indikation. Außerdem befindet sich THC in der klinischen Erprobungsphase für die Behandlung von Glaukom und Autoimmunerkrankungen, wie Multipler Sklerose, Morbus Crohn oder Colitis Ulcerosa. Cannabidiol (CBD) wirkt krampf- und angstlösend, entzündungshemmend, gegen Übelkeit, sowie Augeninnendruck-senkend.

Eine Aufgabe der vorliegenden Erfindung ist es, wirksame und nebenwirkungsfreie Medikamente auf Basis von Cannabinoiden zur parenteralen Verabreichung zur Verfügung zu stellen, die über einen langen Zeitraum stabil sind und eine hohe Bioverfügbarkeit der Cannabinoide ermöglichen. Dabei ermöglichen Cannabinoide auf pflanzlicher Basis (sogenannte Phyto-Cannabinoide) nicht nur eine sehr kostengünstige und einfache Bereitstellung der arzneilichen Wirkstoffe, sondern auch eine hohe Compliance der Patienten.

Überraschenderweise wurde gefunden, dass eine Öl-in-Wasser-Emulsion, die Cannabinoide in der Ölphase aus sehr kleinen Tröpfchen enthält, zu einer verbesserten Stabilität der Öl-in-Wasser-Emulsion führen und die darin enthaltenen Cannabinoide verbessert aufgenommen werden können und zu weniger Nebenwirkungen beim Patienten führen.

Daher bezieht sich die beanspruchte Erfindung in einem Aspekt auf eine Öl-in-Wasser-Emulsion zur parenteralen Verabreichung umfassend
(a) wenigstens eine Ölphase umfassend
   wenigstens ein Cannabinoid und wenigstens ein Öl
   und
(b) wenigstens eine wässrige Phase,
wobei die Öl-in-Wasser-Emulsion Tröpfchen mit einer mittleren Tröpfchengröße im Bereich von ≥ 50 nm bis ≤ 500 nm, gemessen durch Photonenkorrelationsspektroskopie, aufweist und einen pFAT₅-Wert ≤ 0.05 Volumen-%, bezogen auf das gesamte Volumen aller Tröpfchen, aufweist.

Der Ausdruck "Ölphase" im Sinne der vorliegenden Erfindung bezieht sich auf eine Phase innerhalb einer Öl-in-Wasser-Emulsion, die aus Öl und/oder lipidhaltigen Flüssigkeiten besteht. Diese Phase bildet die innere oder disperse Phase der Emulsion, das heißt, sie besteht aus Tröpfchen von Öl oder Lipiden, die in der kontinuierlichen wässrigen Phase verteilt sind.

Der Ausdruck "wässrige Phase" bezieht sich auf den Teil einer Emulsion, der aus Wasser oder einer wasserhaltigen Flüssigkeit besteht. In einer Öl-in-Wasser-Emulsion bildet die wässrige Phase die äußere oder kontinuierliche Phase, das heißt, sie umgibt die Öltröpfchen, die in der Emulsion dispergiert sind.

Im Sinne der vorliegenden Erfindung bezeichnet der Ausdruck "Tröpfchen" Öltropfen, das heißt, aus wenigstens einem Öl bestehende Tropfen und/oder öl- und/oder lipidhaltige Tropfen, die die innere oder disperse Phase der Öl-in-Wasser-Präemulsion und/oder Öl-in-Wasser-Emulsion bilden. Die Öl-in-Wasser-Präemulsion zeichnet sich dabei durch eine breitere Tröpfchengrößenverteilung und/oder durch Tropfen größeren Durchmessers, insbesondere größeren mittleren Durchmessers, aus als die Öl-in-Wasser-Emulsion.

Die Photonenkorrelationsspektroskopie (PCS), auch dynamische Lichtstreuung (DLS) genannt, ist eine analytische Methode zur Bestimmung der Größenverteilung von kleinen Tröpfchen in Suspensionen oder Emulsionen. Bei dieser Technik wird die Brownsche Bewegung der Partikel durch Messung der zeitlichen Intensitätsschwankungen des von einem Laserstrahl gestreuten Lichts analysiert. Diese Intensitätsschwankungen entstehen durch Interferenzen des gestreuten Lichts, die von der Diffusion der Partikel beeinflusst werden. Durch die Berechnung der Autokorrelationsfunktion des Streulichtsignals kann der Diffusionskoeffizient der Partikel ermittelt werden, aus dem sich die mittlere Tröpfchengröße ableiten lässt.

Der pFAT₅-Wert (percentage of Fat above 5 microns) ist ein Maß für den Anteil der Fetttröpfchen in einer Emulsion, gemessen in Volumen-%, die einen Durchmesser von mehr als 5 Mikrometern aufweisen. Dieser Wert ist besonders wichtig in der parenteralen Ernährung, da größere Fetttröpfchen das Risiko von Fettembolien erhöhen können. Ein niedriger pFAT₅-Wert zeigt an, dass die Emulsion gut homogenisiert ist und nur wenige große Fetttröpfchen enthält. Die Bestimmung der pFAT₅-Werte erfolgt gemäß den Vorgaben der United States Pharmacopeia (USP) <729>, die die Lichtblockadetechnologie verwendet. Diese Methode ermöglicht die präzise Messung der Partikelgrößenverteilung in lipidbasierten Emulsionen, indem die Lichtdurchlässigkeit durch die Fetttröpfchen erfasst wird. Eine Emulsionsprobe wird dabei durch ein Messgerät geleitet, das die Reduktion der Lichtintensität detektiert und in elektrische Signale umwandelt, die proportional zur Größe der Tröpfchen sind. Basierend auf diesen Daten wird der Anteil der Fetttröpfchen mit einem Durchmesser von über 5 Mikrometern berechnet, um den pFAT₅-Wert zu bestimmen.

In einer bevorzugten Ausführungsform liegt die mittlere Tröpfchengröße im Bereich von ≥ 100 nm bis ≤ 400 nm, besonders bevorzugt im Bereich von ≥ 150 nm bis ≤ 300 nm, ganz besonders bevorzugt im Bereich von ≥ 200 nm bis ≤ 280 nm, gemessen durch Photonenkorrelationsspektroskopie.

Im Sinne der vorliegenden Erfindung bezieht sich parenterale Verabreichung auf die Verabreichung von Medikamenten oder therapeutischen Substanzen durch Injektion oder Infusion, wobei der Magen-Darm-Trakt umgangen wird. Diese Methode ermöglicht eine direkte Einführung des Wirkstoffs in den Blutkreislauf oder spezifische Gewebe, was eine schnellere und kontrolliertere therapeutische Wirkung gewährleistet. Die Injektion oder Infusion kann intravenös, intramuskulär, subkutan, intrathekal oder intraarteriell erfolgen.

In einer bevorzugten Ausführungsform weist die erfindungsgemäß beanspruchte Öl-in-Wasser Emulsion einen pFAT₅-Wert ≤ 0,03 Volumen-% auf, besonders bevorzugt ≤ 0,01 Volumen-%, bezogen auf das gesamte Volumen aller Tröpfchen. Ganz besonders bevorzugt liegt der pFATs Wert im Bereich von ≥ 0,001 Volumen-% bis ≤ 0,01 Volumen-%, bezogen auf das gesamte Volumen aller Tröpfchen, liegt.

In einer bevorzugten Ausführungsform umfasst die erfindungsgemäß beanspruchte Öl-in-Wasser Emulsion ein Öl, wobei das Öl ausgewählt wird aus einer Gruppe bestehend aus pflanzlichen Ölen, Omega-3-Triglyceriden, mittelkettigen Triglyceriden, und deren Kombinationen.

Die Ölphase wird unter Verwendung wenigstens eines Öls hergestellt. Bevorzugte Öle umfassen pflanzliche Öle, Omega-3-Triglyceride, mittelkettige Triglyceride und deren Kombinationen. Pflanzliche Öle, auch als Pflanzenöle bekannt, sind fette Öle, die aus den Samen, Früchten oder anderen Pflanzenteilen extrahiert werden. Bevorzugte pflanzliche Öle werden aus der Gruppe bestehend aus Olivenöl, Sonnenblumenöl, Sojaöl, Rapsöl, Maisöl, Sesamöl, Avocadoöl, Traubenkernöl, Walnussöl, Kokosöl, Mandelöl, Jojobaöl, Hanfsamenöl und Leinöl, besonders bevorzugt ist das pflanzliche Öl Sojaöl und/oder Olivenöl ausgewählt. Sojaöl ist ein pflanzliches Öl, das aus den Samen der Sojabohne (Glycine max) gewonnen wird. Es ist reich an mehrfach ungesättigten Fettsäuren, insbesondere Linolsäure (18:2 ω-6), während sein Gehalt an ω-3-Fettsäuren, hauptsächlich α-Linolensäure (18:3 ω-3), gering ist. Olivenöl ist ein pflanzliches Öl, das durch das Pressen von Oliven (Olea europaea) gewonnen wird. Es besteht hauptsächlich aus einfach ungesättigten Fettsäuren, insbesondere Ölsäure (18:1 ω-9), und enthält auch eine geringe Menge an mehrfach ungesättigten Fettsäuren. Olivenöl ist bekannt für seinen hohen Gehalt an Antioxidantien, einschließlich Vitamin E und Polyphenolen.

Omega-3-Triglyceride sind Fettsäuren, die in Form von Triglyceriden vorliegen und reich an Omega-3-Fettsäuren sind. Diese Fettsäuren sind essenziell für den menschlichen Körper und spielen eine wichtige Rolle bei der Funktion von Zellmembranen und im Entzündungsstoffwechsel. Bevorzugte Omega-3-Triglyceride werden ausgewählt aus der Gruppe bestehend aus Fischöl, Algenöl, Leinsamenöl, Chiaöl, Hanföl und Walnussöl. Bevorzugte Fischöle werden ausgewählt aus der Gruppe bestehend aus Sardinenöl, Lachsöl, Heringöl und Makrelenöl. Diese Fischöle sind besonders reich an Eicosapentaensäure (EPA) und Docosahexaensäure (DHA), zwei wichtigen Omega-3-Fettsäuren.

In einer bevorzugten Ausführungsform enthält die erfindungsgemäß beanspruchte mittelkettige Triglyceride, die ausgewählt werden aus der Gruppe bestehend aus Triglyceriden von Capronsäure, Triglyceriden von Caprylsäure , Triglyceriden von Caprinsäure und Triglyceriden von Laurinsäure.

In einer bevorzugten Ausführungsform liegt die Ölphase in einer Menge von ≥ 3,0 bis ≤ 40 Gew.-%, bevorzugt in einer Menge von ≥ 5,0 bis ≤ 30 Gew.-%, besonders von ≥ 10,0 bis ≤ 25 Gew.-%, bezogen auf die gesamte Menge der Öl-in-Wasser-Emulsion, vor. In einer bevorzugten Ausführungsform liegt die wässrige Phase in einer Menge von ≥ 60 bis ≤ 97 Gew.-%, bevorzugt in einer Menge von ≥ 70 bis ≤ 95 Gew.-%, besonders von ≥ 72 bis ≤ 90 Gew.-%, bezogen auf die gesamte Menge der Öl-in-Wasser-Emulsion, vor.

Cannabinoide entfalten ihre Wirkung im menschlichen Körper hauptsächlich durch die Interaktion mit dem Endocannabinoid-System (ECS), das aus Endocannabinoiden und deren Rezeptoren (CB1 und CB2) besteht. Der CB1-Rezeptor ist vorwiegend im Zentralnervensystem zu finden und beeinflusst Prozesse wie Schmerzempfinden, Gedächtnis und Appetitregulation. Der CB2-Rezeptor ist hauptsächlich in Zellen des Immunsystems vorhanden und spielt eine Rolle bei Entzündungs- und Immunreaktionen.

Tetrahydrocannabinol (THC), das bekannteste Cannabinoid, bindet direkt an diese Rezeptoren und verursacht psychoaktive Effekte sowie eine Schmerzlinderung. Cannabidiol (CBD) hingegen interagiert weniger direkt mit den Cannabinoid-Rezeptoren und wirkt durch Modulation anderer Signalwege, was zu entzündungshemmenden, anxiolytischen und antiepileptischen Effekten führt. Weitere Cannabinoide wie Cannabigerol (CBG) und Cannabichromen (CBC) tragen ebenfalls zur komplexen pharmakologischen Wirkung bei, indem sie unterschiedliche Rezeptoren und Signalwege beeinflussen.

Die Öl-in-Wasser-Emulsion gemäß der beanspruchten Erfindung enthält cannabinoid-artige Verbindungen, die zu den 12 Haupttypen von Cannabinoiden gehören. Diese umfassen tetrahydrocannabinol-artige, cannabidiol-artige, cannabigerol-artige, cannabichromen-artige, cannabicyclol-artige, cannabielsoin-artige, cannabitriol-artige, cannabinol-artige, cannabinodiol-artige, cannabicitran-artige, cannabichromanon-artige und Isocannabinoide. Cannabinoid-artige Verbindungen sind chemische Substanzen, die entweder in der Cannabispflanze vorkommen (Phytocannabinoide) oder synthetisch hergestellt werden können (synthetische Cannabinoide).

In einer bevorzugten Ausführungsform umfasst die Öl-in-Wasser-Emulsion gemäß der beanspruchten Erfindung wenigstens eine Cannabinoid, das ausgewählt wird aus der Gruppe bestehend aus Tetrahydrocannabinol (THC), Δ8-Tetrahydrocannabinol (Δ8-THC), Δ9-Tetrahydrocannabivarin (Δ9-THDV), Cannabidiol (CBD), Cannabinol (CBN), Cannabigerol (CBG), Cannabidivarin (CBDV), Cannabichromen (CBC), Cannabicyclol (CBL), Cannabielsoin (CBE), Cannabitriol (CBT), Cannabinodiol (CBND), Cannabicitran (CBT), Cannabichromanon (CBCN), Tetrahydrocannabinolsäure (THCA), Tetrahydrocannabivarin (THCV) und deren Derivaten.

Cannabinoide teilen einige gemeinsame physikalische und chemische Eigenschaften, die ihre Formulierung und Anwendung beeinflussen. Eine der hervorstechendsten Eigenschaften ist ihre hohe Lipophilie, was bedeutet, dass sie sich gut in Ölen und Fetten lösen, aber schlecht in Wasser. Dies ist auf die chemische Struktur der Cannabinoide zurückzuführen, die überwiegend aus Kohlenstoff- und Wasserstoffatomen besteht und somit hydrophob ist.

Die Löslichkeit in Öl ermöglicht die Formulierung von Cannabinoid-Produkten in Form von Öl-in-Wasser-Emulsionen, bei denen die Cannabinoide in der Ölphase gelöst sind. Diese Emulsionen bieten Vorteile wie eine verbesserte Bioverfügbarkeit und eine kontrollierte Freisetzung der Wirkstoffe. Darüber hinaus weisen Cannabinoide einen relativ niedrigen Schmelzpunkt auf, was ihre Verarbeitung bei moderaten Temperaturen erleichtert. Chemisch gesehen sind Cannabinoide relativ stabil, können jedoch durch Licht, Hitze und Sauerstoff abgebaut werden, weshalb sie oft in dunklen, luftdichten Behältern gelagert werden müssen, um ihre Wirksamkeit zu bewahren.

In einer bevorzugten Ausführungsform weist das wenigstens eine Cannabinoid einen log-P-Wert im Bereich von ≥ 6,00 bis ≤ 8,00 auf, der dessen gute Löslichkeit in der Ölphase widerspiegelt. Besonders bevorzugt wird das wenigstens eine Cannabinoid ausgewählt aus der Gruppe bestehend aus Tetrahydrocannabinol (THC), Δ8-Tetrahydrocannabinol (Δ8-THC), Δ9-Tetrahydrocannabivarin (Δ9-THDV), Cannabidiol (CBD), Cannabinol (CBN), Cannabigerol (CBG), Cannabinodiol (CBND), Cannabicitran (CBT) und Cannabichromanon (CBCN). Ganz besonders bevorzugt ist das wenigstens eine Cannabinoid Tetrahydrocannabinol (THC) oder Cannabidiol (CBD). In einer bevorzugten Ausführungsform liegt das wenigstens eine Cannabinoid in einer Menge von ≥ 0,1 bis ≤ 10 Gew.-%, besonders bevorzugt in einer Menge von ≥ 0,5 bis ≤ 8,0 Gew.-%, ganz besonders bevorzugt in einer Menge von ≥ 0,5 bis ≤ 5,0 Gew.-%, noch weiter bevorzug in einer Menge von ≥ 0,5 bis ≤ 2,0 Gew.-%, bezogen auf die gesamte Menge der Öl-in-Wasser-Emulsion, vor.

Bei der Herstellung der Öl-in-Wasser-Emulsion werden Glycerol als auch Natriumhydroxid in der wässrigen Phase gelöst. Die wässrige Phase und die Ölphase werden auf eine Temperatur im Bereich von 20 °C bis 70 °C erwärmt, um die Emulgatoren optimal in der wässrigen Phase zu lösen und so die Stabilität der Emulsion zu gewährleisten. Die Ölphase wird nicht nur unter Verwendung wenigstens eines Öls, sondern auch unter Verwendung wenigstens eines Emulgators hergestellt. Emulgatoren sind entscheidend, um die Stabilität der Emulsion zu gewährleisten, indem sie die Oberflächenspannung zwischen der Ölphase und der wässrigen Phase verringern und die Bildung stabiler Tröpfchen fördern.

In einer bevorzugten Ausführungsform umfasst die Öl-in-Wasser-Emulsion weiterhin wenigstens einen Emulgator, der ausgewählt wird aus der Gruppe bestehend aus Ei-Lecithin, Sojalecithin und deren Kombinationen. Ei-Lecithin, auch als Lecithin aus Eigelb bekannt, ist eine Mischung von Phospholipiden, die aus Eiern gewonnen wird. Ei-Lecithin enthält hauptsächlich Phosphatidylcholin, Phosphatidylethanolamin und Phosphatidylinositol. Soja-Lecithin ist ein Gemisch von Phospholipiden, das aus Sojabohnen gewonnen wird. Soja-Lecithin enthält hauptsächlich Phosphatidylcholin, Phosphatidylethanolamin und Phosphatidylinositol. Bevorzugt liegt der wenigstens eine Emulgator in einer Menge von ≥ 0,1 bis ≤ 7,0 Gew.-%, besonders bevorzugt in einer Menge von ≥ 0,2 bis ≤ 5,0 Gew.-%, ganz besonders bevorzugt in einer Menge von ≥ 0,5 bis ≤ 2,0 Gew.-%, bezogen auf die gesamte Menge der Öl-in-Wasser-Emulsion, vor.

In einer bevorzugten Ausführungsform weist die Öl-in-Wasser-Emulsion gemäß der beanspruchten Erfindung weiterhin wenigstens einen Co-Emulgator auf, der ausgewählt wird aus der Gruppe bestehend aus Fettsäuren wie Ölsäure, Polyoxyethylen-basierte Verbindungen wie Polyoxyethylen-(20)-sorbitan-monooleat (Polysorbat 80), Sorbitanmonostearat, und deren Kombinationen umfasst. Fettsäuren sind Carbonsäuren mit einer langen aliphatischen Kette, die entweder gesättigt oder ungesättigt sein kann. Sie bestehen aus einer Carboxylgruppe (-COOH) und einer Kohlenwasserstoffkette, die normalerweise zwischen 12 und 28 Kohlenstoffatome umfasst. Typische Beispiele für Fettsäuren sind Stearinsäure (gesättigt), Ölsäure (einfach ungesättigt) und Linolsäure (mehrfach ungesättigt). Polyoxyethylen-basierte Verbindungen sind chemische Substanzen, die auf Polyethylenoxid (PEO) basieren und als wichtige Klasse von nichtionischen Tensiden und Emulgatoren dienen. Diese Verbindungen werden durch die Polymerisation von Ethylenoxid gebildet und enthalten wiederholende -CH2CH2O- Einheiten in ihrer Struktur. Typische Beispiele für polyoxyethylen-basierte Verbindungen sind Polyoxyethylen-(20)-sorbitan-monooleat (Polysorbat 80), Polyoxyethylen-glyceroltrioleat und Polyoxyethylenstearat. Bevorzugt liegt der wenigstens eine Co-Emulgator in einer Menge von ≥ 0,01 bis ≤ 5,0 Gew.-%, besonders bevorzugt in einer Menge von ≥ 0,01 bis ≤ 1,0 Gew.-%, bezogen auf die gesamte Menge der Öl-in-Wasser-Emulsion, vor. Bevorzugt liegt Ölsäure in einer Menge von ≥ 0,01 bis ≤ 0,10 Gew.-%, besonders bevorzugt in einer Menge von ≥ 0,01 bis ≤ 0,05 Gew.-%, bezogen auf die gesamte Menge der Öl-in-Wasser-Emulsion, vor.

In einer bevorzugten Ausführungsform weist die Öl-in-Wasser-Emulsion gemäß der beanspruchten Erfindung einen pH-Wert auf, der im Bereich von ≥ 6,0 bis ≤ 8,5, bevorzugt im Bereich von ≥ 7,5 bis ≤ 8,5, liegt. Der pH-Wert einer der Öl-in-Wasser-Emulsion wird mittels eines pH-Meters gemessen, welches eine Elektrode verwendet, um die Wasserstoffionenkonzentration in der Lösung zu bestimmen. Der genaue Messprozess umfasst die Kalibrierung des pH-Meters mit Standardpufferlösungen vor der Messung, um genaue Ergebnisse zu gewährleisten. Nach der Kalibrierung wird die Elektrode in die Emulsion getaucht und der pH-Wert direkt abgelesen. Die Öl-in-Wasser-Emulsion gemäß der beanspruchten Erfindung wird auf den richtigen pH-Wert eingestellt, um ihre Stabilität und Wirksamkeit zu gewährleisten. Dazu werden pharmazeutisch zugelassene Basen wie Natriumhydroxid verwendet. Diese Basen helfen dabei, den pH-Wert der Emulsion im gewünschten Bereich zu halten, was entscheidend für die optimale Mischung und Stabilität der Öl- und Wasserphasen ist.

In einer bevorzugten Ausführungsform weist die Öl-in-Wasser-Emulsion gemäß der beanspruchten Erfindung eine Säurezahl auf, die im Bereich von ≥ 0.01 mg KOH/g bis ≤ 2 mg KOH/g liegt. Zur Bestimmung der Säurezahl in der erfindungsgemäß beanspruchten Öl-in-Wasser-Emulsion werden Methoden angewendet, die in der pharmazeutischen Industrie akzeptiert und dem Fachmann bekannt sind. Diese Methoden beinhalten typischerweise die Titration mit einer standardisierten Kaliumhydroxid-Lösung, bei der die Menge an freien Fettsäuren in der Probe durch Neutralisation mit KOH gemessen wird. Der Endpunkt der Titration wird mithilfe eines Indikators wie Phenolphthalein ermittelt, der eine Farbänderung anzeigt. Diese etablierten Verfahren sind standardisiert und gewährleisten präzise und reproduzierbare Messungen, die für die Qualitätskontrolle und Sicherstellung der Produktstabilität in der pharmazeutischen Industrie unerlässlich sind. Durch die Einhaltung dieser anerkannten Methoden wird sichergestellt, dass die Ergebnisse zuverlässig und den hohen Anforderungen der Branche entsprechend sind.

In einer bevorzugten Ausführungsform weist die Öl-in-Wasser-Emulsion gemäß der beanspruchten Erfindung eine Peroxidzahl von ≤ 5 meq O₂/kg auf. Zur Bestimmung der Peroxidzahl in der erfindungsgemäß beanspruchten Öl-in-Wasser-Emulsion werden Methoden verwendet, die in der pharmazeutischen Industrie anerkannt und dem Fachmann bekannt sind. Diese Methoden umfassen typischerweise iodometrische Titrationsverfahren, bei denen die Menge an Peroxiden durch ihre Reaktion mit Kaliumiodid und die anschließende Titration des freigesetzten Iods mit Natriumthiosulfat gemessen wird. Diese Verfahren sind standardisiert und ermöglichen präzise und reproduzierbare Messungen, die essentiell für die Qualitätskontrolle und Sicherstellung der Stabilität pharmazeutischer Produkte sind. Die Einhaltung dieser etablierten Methoden gewährleistet, dass die Ergebnisse zuverlässig sind und den hohen Anforderungen der pharmazeutischen Industrie entsprechen.

In einer bevorzugten Ausführungsform sind die Öl-in-Wasser-Emulsion gemäß der vorliegenden Erfindung auch nach 24 Monaten Lagerung bei Raumtemperatur und 60 % relativer Luftfeuchtigkeit noch so stabil und zeigen keine Anzeichen einer Phasentrennung. Die erfindungsgemäß beanspruchten Öl-in-Wasser-Emulsionen zeichnen sich durch eine besonders hohe Stabilität aus, die auf die feine mittlere Teilchengröße von weniger als 500 nm und die kontrollierte Prozessführung zurückzuführen ist. Durch den Einsatz des erfindungsgemäßen Herstellungsverfahrens unter Einsatz von Hochdruckhomogenisatoren wird eine sehr feine Verteilung der Öltröpfchen erreicht. Die mittlere Teilchengröße von weniger als 500 nm sorgt dafür, dass die Öltröpfchen gleichmäßig in der wässrigen Phase verteilt bleiben und das Risiko der Koaleszenz, also des Zusammenflusses der Tröpfchen, minimiert wird. Diese feine Tröpfchengröße trägt entscheidend zur Stabilität der Emulsion bei, da kleinere Tröpfchen weniger dazu neigen, sich zu größeren Tropfen zusammenzuschließen und somit eine Phasentrennung zu verursachen. Die Stabilität der Emulsionen wird anhand der Phasentrennung bestimmt. Eine stabile Emulsion zeigt keine oder nur sehr geringe Phasentrennung über einen längeren Zeitraum. Im Gegensatz dazu ist eine Emulsion, die zur Phasentrennung neigt, instabil und verliert ihre einheitliche Struktur.

In einer bevorzugten Ausführungsform weist die Öl-in-Wasser-Emulsion gemäß der beanspruchten Erfindung eine Osmolalität im Bereich von ≥ 150 bis 500 mOsm/kg auf. Zur Bestimmung der Osmolalität der erfindungsgemäß beanspruchten Öl-in-Wasser-Emulsionen werden Methoden verwendet, die in der pharmazeutischen Industrie akzeptiert und dem Fachmann bekannt sind. Eine häufig verwendete Methode ist die Gefrierpunktosmometrie. Diese Methode misst die Absenkung des Gefrierpunkts der Lösung, die proportional zur Anzahl der gelösten Teilchen ist. Die Probenvorbereitung erfolgt durch Einbringen der Emulsionsprobe in das Probenfach des Osmometers. Während der Messung senkt das Gerät die Temperatur der Probe bis unter den Gefrierpunkt, und ein Rührmechanismus induziert das Gefrieren. Die Temperatur, bei der die Probe gefriert, wird gemessen und die Osmolalität aus der Gefrierpunkterniedrigung berechnet, typischerweise in Osmol pro Kilogramm (Osm/kg) angegeben. Die Gefrierpunktosmometrie ist eine bewährte, zuverlässige und präzise Technik, die schnelle und genaue Ergebnisse liefert, ohne die Probe zu verändern oder zu zerstören. Durch die Anwendung dieser etablierten Methode wird sichergestellt, dass die Bestimmung der Osmolalität den hohen Anforderungen der pharmazeutischen Industrie entspricht und die Ergebnisse zuverlässig und reproduzierbar sind. Die Osmolalität der erfindungsgemäß beanspruchten Öl-in-Wasser-Emulsionen kann durch die Zugabe von isotonisch wirkenden Mitteln eingestellt werden, um eine optimale Verträglichkeit bei parenteraler Verabreichung zu gewährleisten. Zu den gängigen isotonisch wirkenden Mitteln gehören Glycerol, Glucose und Polyethylenglykol (PEG).

In einer bevorzugten Ausführungsform umfasst die Öl-in-Wasser-Emulsion gemäß der beanspruchten Erfindung weiterhin wenigstens ein Konservierungsmittel ausgewählt aus der Gruppe bestehend aus EDTA, Benzylalkohol, Chlorbutanol, Methylparaben, Propylparaben und deren Kombinationen. Die erfindungsgemäß verwendeten Konservierungsmittel erfüllen strenge regulatorische Anforderungen, wie sie von Gesundheitsbehörden wie der FDA (Food and Drug Administration) und EMA (European Medicines Agency) festgelegt sind. Die Konservierungsmittel werden in den jeweiligen Pharmakopöen gelistet und sind für den Einsatz in parenteralen Produkten zugelassen. In einer bevorzugten Ausführungsform liegt das wenigstens eine Konservierungsmittel in einer Menge von ≥ 0,1 bis ≤ 5,0 Gew.-%, bevorzugt in einer Menge von ≥ 0,1 bis ≤ 2,0 Gew.-%, bezogen auf die gesamte Menge der Öl-in-Wasser-Emulsion, vor.

In einer bevorzugten Ausführungsform umfasst die Öl-in-Wasser-Emulsion gemäß der beanspruchten Erfindung weiterhin wenigstens einen Puffer ausgewählt aus der Gruppe bestehend aus Phosphatpuffern, Tris-Puffer und deren Kombinationen.

Die erfindungsgemäß beanspruchte Öl-in-Wasser-Emulsion ist zur parenteralen Verabreichung vorgesehen und kann in verschiedenen Verabreichungsschemata angewendet werden, um die Behandlung oder Prävention der jeweiligen Krankheit zu unterstützen. Alle gängigen Verabreichungsschemata für Öl-in-Wasser-Emulsionen sind möglich, um die therapeutischen Bedürfnisse der Patienten optimal zu erfüllen. Bevorzugt wird die Öl-in-Wasser-Emulsion gemäß der beanspruchten Erfindung einmal täglich, zweimal täglich, dreimal täglich oder viermal täglich verabreicht oder einmal wöchentlich, alle drei Tage oder jeden zweiten Tag. Neben den intermittierenden Verabreichungsschemata wird bei der parenteralen Verabreichung auch eine Dauerinfusion bevorzugt. Bei einer Dauerinfusion wird die Öl-in-Wasser-Emulsion kontinuierlich über einen längeren Zeitraum, typischerweise über mehrere Stunden oder Tage, direkt in den Blutkreislauf des Patienten infundiert. Diese Methode ermöglicht eine gleichmäßige und konstante Verabreichung des Wirkstoffs, was insbesondere bei Erkrankungen mit hohem Bedarf an stabilen Wirkstoffspiegeln im Blut vorteilhaft ist. Die Dauerinfusion wird mithilfe einer Infusionspumpe durchgeführt, die die Emulsion mit einer kontrollierten und konstanten Flussrate in den Körper des Patienten einbringt. Diese Art der Verabreichung ist besonders geeignet für Patienten, die eine kontinuierliche therapeutische Wirkung benötigen, und stellt sicher, dass der Wirkstoff gleichmäßig verteilt und über den gesamten Infusionszeitraum wirksam bleibt.

Im Sinne der vorliegenden Erfindung umfasst Behandlung alle Maßnahmen, die darauf abzielen, die Symptome einer Erkrankung zu lindern, die Progression der Krankheit zu verlangsamen oder zu stoppen, und die Lebensqualität des Patienten zu verbessern. Diese Maßnahmen beinhalten sowohl die präventive als auch die therapeutische Anwendung von Medikamenten auf Basis von Cannabinoiden.

Cannabinoide werden vorwiegend oral oder pulmonal appliziert, was eine therapeutisch wichtige präzise Dosierung kaum möglich macht. Zudem erfahren enteral verabreichte Arzneistoffe den First-Pass-Effekt, sodass neben der schwankenden Resorptionsquote auch der Metabolismus in der Leber zur Verminderung der Bioverfügbarkeit führt. Den idealen Applikationsweg stellt somit die parenterale Gabe der Cannabinoide dar, da hierbei reproduzierbar 100 % des Arzneistoffes dem Körper zur Verfügung stehen.

Aufgrund des lipophilen Charakters dieser Stoffgruppe können Cannabinoide nicht als wässrige Lösung formuliert werden, sondern benötigen ein Trägersystem, in dem der Arzneistoff solubilisiert wird. Ein solches "Drug-Delivery-System" stellen Emulsionen dar, wobei der Arzneistoff in der inneren lipophilen Phase gelöst wird.

Die vorliegende Erfindung beschreibt eine Emulsion bestehend aus einer Ölphase aus pflanzlichen Ölen und/oder halbsynthetischen mittelkettigen Triglyceriden und einer äußeren wässrigen Phase.

Eine Formulierung als Emulsion ermöglicht eine präzise und reproduzierbare Dosierung des Cannabinoids, was den therapeutischen Einsatz in unterschiedlichen medizinischen Indikationen erst ermöglicht.

Pharmakologische Anwendungsbereiche der erfindungsgemäßen Cannabinoid-Emulsionen:
Anwendung der Cannabinoid-Emulsion als:
1. Analgetikum
2. Antinozizeptikum
3. Spasmolytikum
4. Antiemetikum
5. Sedativum
6. Appetitanreger (Orexigen) /-unterdrücker (Anorektika)
7. Appetitzügler
8. Antikonvulsivum
9. Neuroprotektor / Neuroprotektivum
10. Antipsychotikum
11. Anxiolytikum
12. Antiphlogistikum / Antiinflammatorikum
13. Antirheumatikum
14. Myotonolytikum
15. Neuroleptikum
16. Antiepileptikum
17. Osteostimulanz
18. Antibiotikum
19. Immunmodulation
20. Blutdrucksenkung/-steigerung
21. Bradykardie/Tachykardie

Belegte medizinische Indikationen umfassen:
Chronische Schmerzen (insbesondere bei Krebserkrankungen und -therapie), Multiple Sklerose, Übelkeit, Erbrechen, Appetitanregung und -unterdrückung, Angstzustände, Schlafstörungen, Fibromyalgie, Gilles-de-la-Tourette-Syndrom, therapieresistentes Glaukom, rheumatoide Arthritis, entzündliche Darmerkrankungen (Morbus Crohn, Colitis ulcerosa). psychotische Störungen (Schizophrenie), Epilepsie und Bewegungsstörungen, Schlaganfall, Parkinson, Migräne, Rückenmarksverletzungen, periphere Neuropathie und andere neurogene Schmerzen, sowie Aufmerksamkeitsdefizitstörung (ADS) und posttraumatische Belastungsstörung (PTBS).

Ferner können die erfindungsgemäßen Emulsionen zur Behandlung von Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hämatologische Neoplasien, Haarzell-Leukämie, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals- Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome, u. osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs eingesetzt werden.

Auch zur Behandlung von neurodegenerativen Erkrankungen ausgewählt aus der Gruppe umfassend Alzheimer Erkrankung, Parkinson'sche Krankheit, Hemiatrophie-Hemiparkinson, Parkinsonsyndrom, Huntington'sche Krankheit, amyotrophe laterale Sklerose, Demenz, Demenz bedingt durch AIDS, Retinitis Pigmentosa, muskuläre Atrophie, muskuläre Atrophie der Wirbelsäule, paraneoplastische zerebellare Degeneration (PCD), zerebellare Atrophie, extrapyramidale Atrophie, Multi-Systematrophie, Progressive Supranucleäre Blickparese, Ataxie, Multiple Sklerose, Phakomatosen, FXTAS (fragile X-associated tremor/ataxia syndrome) auch bezeichnet als das fragile X-Syndrom, progressive supranukleäre Lähmung (PSP: progressive supranuclear palsy), striatonigrale Degeneration (SND), olivopontozerebellare Degeneration (OPCD), Shy Drager syndrome (SDS), kortiko-basale Degeneration, Lewy-Körperchen-Demenz, Lewy-Körperchen Krankheit, striato-nigrale Degeneration (SND), idiopathische orthostatische Hypotension (IOH), Multisystematrophien frontotemporale Demenz, Lytico-Bodig Krankheit (Parkinsonismus-Demenz-amyotrophe Lateralsklerose), progressive pallidale Atrophie, Hallervorden-Spatz Erkrankung, x-Chromosom verknüpfte Dystonie (Morbus Lubag), mitochondriale Zytopathie mit striataler Nekrose, Neuroakanthocytose, Restless Leg Syndrom, Wilson'sche Krankheit als auch multiple System-Atrophie (MSA) können die erfindungsgemäßen Emulsionen verwendet werden.

Auch zur Behandlung einer der folgenden seltenen Krankheiten können die erfindungsgemäßen Emulsionen eingesetzt werden:
ACTH Deficiency; AIDS (Acquired Immune Deficiency Syndrome); AIDS Dysmorphic Syndrome; APECED Syndrome; Aarskog Syndrome; Aase Syndrome ; Abetalipoproteinemia; Ablepharon Macrostomia Syndrome; Acanthocheilonemiasis; Acanthocytosis; Acanthosis Nigricans; Achalasia; Achard Thiers Syndrome; Achondrogenesis; Achondroplasia; Acidemia, Isovaleric; Acidemia; Methylmalonic; Acidemia, Propionic; Acne Rosacea; Acoustic Neuroma; Acquired Aplastic Anemia; Acrocallosal Syndrome, Schinzel Type; Acrodermatitis Enteropathica; Acrodysostosis; Acromegaly; Acromesomelic Dysplasia; Acromicric Dysplasia; Acute Respiratory Distress Syndrome; Adams Oliver Syndrome; Addison's Disease; Adenoid Cystic Carcinoma; Adenylosuccinate Lyase Deficiency; Adie Syndrome; Adrenal Hyperplasia, Congenital (General); Adrenoleukodystrophy; Afibrinogenemia, Congenital; Agammaglobulinemias, Primary; Agenesis of Corpus Callosum; Agnosia, Primary Visual; Agranulocytosis, Acquired; Ahumada-Del Castillo Syndrome; Aicardi Syndrome; Alagille Syndrome; Albinism; Alexander Disease; Alkaptonuria; Allan Herndon Syndrome; Alopecia Areata; Alpers Disease; Alpha-1-Antitrypsin Deficiency; Alpha-Mannosidosis; Alport Syndrome; Alstrom Syndrome; Alternating Hemiplegia of Childhood; Alveolar Capillary Dysplasia; Alveolar Soft Part Sarcoma; Alveolitis, Extrinsic Allergic; Alzheimer's Disease; Ameloblastoma; Amelogenesis Imperfecta; Amenorrhea, Primary; Amenorrhea-Galactorrhea Syndrome; Amniotic Bands; Amyloidosis; Amyotrophic Lateral Sclerosis; Anaphylaxis; Andersen Disease (GSD IV); Androgen Insensitivity Syndrome, Partial; Anemia, Blackfan Diamond; Anemia, Fanconi's; Anemia, Hemolytic, Acquired Autoimmune; Anemia, Hemolytic, Cold Antibody; Anemia, Hemolytic, Warm Antibody; Anemia, Hereditary Nonspherocytic Hemolytic; Anemia, Hereditary Spherocytic Hemolytic; Anemia, Megaloblastic; Anemia, Pernicious; Anemias, Sideroblastic; Anencephaly; Angelman Syndrome; Angioedema, Hereditary; Aniridia; Aniridia Cerebellar Ataxia Mental Deficiency; Ankylosing Spondylitis; Anodontia; Anorexia Nervosa; Anthrax; Antiphospholipid Syndrome; Antisocial Personality Disorder; Antithrombin III Deficiency; Antley Bixler Syndrome; Apert Syndrome; Aplasia Cutis Congenita; Apnea, Infantile; Apnea, Sleep; Apraxia; Arachnoid Cysts; Arachnoiditis; Arginase Deficiency; Argininosuccinic Aciduria; Arnold-Chiari Malformation; Arteriovenous Malformation; Arteritis, Giant Cell; Arteritis, Takayasu; Arthritis, Infectious; Arthritis, Juvenile Rheumatoid; Arthritis, Psoriatic; Arthrogryposis Multiplex Congenita; Asherman's Syndrome; Aspartylglycosaminuria; Asperger's Syndrome; Aspergillosis; Astrocytoma; Astrocytoma, Malignant; Ataxia Telangiectasia; Ataxia with Vitamin E Deficiency; Ataxia, Friedreich's; Ataxia, Hereditary, Autosomal Dominant; Atrial Septal Defects; Atrioventricular Septal Defect; Attention Deficit Hyperactivity Disorder; Atypical Hemolytic Uremic Syndrome; Atypical Mole Syndrome; Autism; Autoimmune Polyendocrine Syndrome Type II; Autoimmune Thyroiditis; Babesiosis; Balantidiasis; Baller Gerold Syndrome; Balo Disease; Bannayan Riley Ruvalcaba Syndrome; Banti's Syndrome; Bardet Biedl Syndrome; Barrett Esophagus; Barth Syndrome; Bartonellosis; Bartter's Syndrome; Batten Disease; Beals Syndrome; Beckwith Wiedemann Syndrome; Behcet's Syndrome; Bejel; Bell's Palsy; Benign Essential Tremor; Bernard Soulier Syndrome; Berylliosis; Best Vitelliform Macular Dystrophy; Biliary Atresia, Extrahepatic; Binswanger's Disease; Bjornstad Syndrome; Bladder Exstrophy-Epispadias-Cloacal Exstrophy Complex; Blastomycosis; Blepharophimosis, Ptosis, Epicanthus Inversus Syndrome; Blepharospasm, Benign Essential; Bloom Syndrome; Blue Diaper Syndrome; Blue Rubber Bleb Nevus; Borjeson Syndrome; Botulism; Bowen Hutterite Syndrome; Bowen's Disease; Bowenoid Papulosis; Brachial Plexus Palsy; Brain Tumors, General; Branchio Oculo Facial Syndrome; Branchio Oto Renal Syndrome; Bronchopulmonary Dysplasia (BPD); Brown Sequard Syndrome; Brown Syndrome; Brucellosis; Bubonic Plague; Budd Chiari Syndrome; Buerger's Disease; Bulimia; Bullous Pemphigoid; Burning Mouth Syndrome; C Syndrome; CHARGE Syndrome; Campomelic Syndrome; Camurati-Engelmann Disease; Canavan Disease; Cancer, Colon; Cancer, Prostate; Cancers, Skin, General; Candidiasis; Carbamyl Phosphate Synthetase Deficiency; Carbohydrate Deficient Glycoprotein Syndrome Type Ia; Carboxylase Deficiency, Multiple; Carcinoid Syndrome; Carcinoma, Renal Cell; Carcinoma, Squamous Cell; Cardiofaciocutaneous Syndrome; Carnitine Deficiency Syndromes; Carnitine Palmitoyltransferase Deficiency; Carnosinemia; Caroli Disease; Carpal Tunnel Syndrome; Carpenter Syndrome; Castleman's Disease; Cat Eye Syndrome; Cat Scratch Disease; Cataract Dental Syndrome; Cataracts; Catel Manzke Syndrome; Caudal Regression Syndrome;Cavernous Malformation; Cayler Syndrome; Celiac Disease; Central Core Disease; Central Hypoventilation Syndrome, Congenital; Cerebellar Agenesis; Cerebellar Degeneration, Subacute; Cerebral Palsy; Cerebro Oculo Facio Skeletal Syndrome; Cerebrocostomandibular Syndrome; Chagas Disease; Chalazion; Chandler's Syndrome; Charcot Marie Tooth Disease; Chediak Higashi Syndrome; Chiari Frommel Syndrome; Chikungunya; Chlamydia; Cholangitis, Primary Sclerosing; Cholecystitis; Cholera; Cholestasis; Chondrocalcinosis, Familial Articular; Chordoma ; Chorea, Sydenham's; Choroideremia; Choroiditis, Serpiginous; Chromosome 10, Distal Trisomy 10q; Chromosome 10, Monosomy 10p; Chromosome 11, Partial Monosomy 11q; Chromosome 11, Partial Trisomy 11q; Chromosome 13, Partial Monosomy 13q; Chromosome 14 Ring; Chromosome 14, Trisomy Mosaic; Chromosome 15 Ring; Chromosome 15, Distal Trisomy 15q; Chromosome 18 Ring; Chromosome 18, Monosomy 18p; Chromosome 18, Tetrasomy 18p; Chromosome 18q- Syndrome; Chromosome 21 Ring; Chromosome 22 Ring; Chromosome 22, Trisomy Mosaic; Chromosome 3, Monosomy 3p2; Chromosome 3, Trisomy 3q2; Chromosome 4 Ring; Chromosome 4, Monosomy 4q; Chromosome 4, Monosomy Distal 4q; Chromosome 4, Partial Trisomy Distal 4q; Chromosome 4, Trisomy 4p; Chromosome 5, Trisomy 5p; Chromosome 6 Ring; Chromosome 6, Partial Trisomy 6q; Chromosome 7, Partial Monosomy 7p; Chromosome 8, Monosomy 8p2; Chromosome 9 Ring; Chromosome 9, Partial Monosomy 9p; Chromosome 9, Tetrasomy 9p; Chromosome 9, Trisomy 9p (Multiple Variants); Chromosome 9, Trisomy Mosaic; Chronic Fatigue Syndrome; Chronic Fatigue Syndrome/Myalgic Encephalomyelitis; Chronic Inflammatory Demyelinating Polyneuropathy; Churg Strauss Syndrome; Ciguatera Fish Poisoning; Cirrhosis, Primary Biliary; Citrullinemia; Cleft Palate and Cleft Lip; Cleidocranial Dysplasia; Clubfoot; Coats' Disease; Cochin Jewish Disorder; Cockayne Syndrome; Coffin Lowry Syndrome; Coffin Siris Syndrome; Cogan Reese Syndrome; Cohen Syndrome; Colitis, Collagenous; Colitis, Ulcerative; Colorado Tick Fever; Common Variable Immunodeficiency; Condyloma; Cone Dystrophy; Congenital Fibrosis of the Extraocular Muscles; Congenital Varicella Syndrome; Conjunctivitis, Ligneous; Conn Syndrome; Conradi Hunermann Syndrome; Conversion Disorder; Cor Triatriatum; Corneal Dystrophies; Cornelia de Lange Syndrome; Corticobasal Degeneration; Costello Syndrome; Cowpox; Craniofrontonasal Dysplasia; Craniometaphyseal Dysplasia; Craniosynostosis, Primary; Creutzfeldt Jakob Disease; Cri du Chat Syndrome; Crigler Najjar Syndrome Type I; Crohn's Disease; Cronkhite-Canada Syndrome; Crouzon Syndrome; Cryoglobulinemia, Essential Mixed; Cryptococcosis; Cushing's Syndrome; Cutaneous T-Cell Lymphomas; Cutis Laxa; Cutis Marmorata Telangiectatica Congenita; Cyclic Vomiting Syndrome; Cystic Fibrosis; Cystic Hygroma; Cysticercosis; Cystinosis; Cystinuria; Cytochrome C Oxidase Deficiency; Cytomegalovirus Infection; DOOR Syndrome; Dandy Walker Malformation; De Barsy Syndrome; De Santis Cacchione Syndrome; Degos Disease; Dejerine Sottas Disease; Dengue Fever; Dentin Dysplasia, Coronal; Dentin Dysplasia, Type I; Dentinogenesis Imperfecta Type III; Depersonalization Disorder; Dercum Disease; Dermatitis Herpetiformis; Dermatitis, Atopic; Dermatitis, Contact; Dermatomyositis; Devic Disease; Dextrocardia with Situs Inversus; DiGeorge Syndrome; Diabetes Insipidus; Diabetes, Insulin Dependent; Diastrophic Dysplasia; Diencephalic Syndrome; Diffuse Idiopathic Skeletal Hyperostosis; Dilatation of the Pulmonary Artery, Idiopathic; Disaccharide Intolerance I; Diverticulitis; Diverticulosis; Down Syndrome; Dracunculosis; Drash Syndrome; Duane Syndrome; Dubin Johnson Syndrome; Dubowitz Syndrome; Duodenal Atresia or Stenosis; Dupuytren's Contracture; Dyggve Melchior Clausen Syndrome; Dysautonomia, Familial; Dyschondrosteosis; Dyskeratosis Congenita; Dyslexia; Dysplasia, Epiphysealis Hemimelica; Dysplasia, Fibrous; Dysthymia; Dystonia; Dystrophy, Asphyxiating Thoracic; Dystrophy, Myotonic; Eales Disease; Ear, Patella, Short Stature Syndrome; Ectodermal Dysplasias; Ectrodactyly Ectodermal Dysplasia Cleft Lip/Palate; Edema, Idiopathic; Ehlers Danlos Syndrome; Eisenmenger Syndrome; Elephantiasis; Ellis Van Creveld Syndrome; Emphysema, Congenital Lobar; Empty Sella Syndrome; Encephalitis, Herpes Simplex; Encephalitis, Japanese; Encephalitis, Rasmussen's; Encephalocele; Endocardial Fibroelastosis; Endocarditis, Infective; Endometriosis; Endomyocardial Fibrosis; Enterobiasis; Eosinophilia Myalgia; Eosinophilic Fasciitis; Epidermal Nevus Syndrome; Epidermolysis Bullosa; Epidermolytic Hyperkeratosis; Epididymitis; Epilepsy; Epitheliopathy, Acute Posterior Multifocal Placoid Pigment; Erdheim Chester Disease; Erysipelas; Erythema Multiforme; Erythroderma desquamativa of Leiner; Erythrokeratodermia with Ataxia; Erythromelalgia; Erythropoietic Protoporphyria; Esophageal Atresia and/or Tracheoesophageal Fistula; Essential Iris Atrophy; Ewing's Sarcoma; Exostoses, Multiple; FG Syndrome; Fabry Disease; Facioscapulohumeral Muscular Dystrophy; Fahr's Disease; Familial Adenomatous Polyposis; Familial Eosinophilic Cellulitis; Familial Juvenile Hyperuricemic Nephropathy; Familial Lipoprotein Lipase Deficiency; Farber's Disease; Fascioliasis; Felty Syndrome; Femoral Facial Syndrome; Fetal Alcohol Syndrome; Fetal Hydantoin Syndrome; Fetal Retinoid Syndrome; Fetal Valproate Syndrome; Fiber Type Disproportion, Congenital; Fibrodysplasia Ossificans Progressiva (FOP); Fibromatosis, Congenital Generalized; Fibromyalgia; Filariasis; Filippi Syndrome; Fitz Hugh Curtis Syndrome; Floating Harbor Syndrome; Focal Dermal Hypoplasia; Forbes Disease; Formaldehyde Poisoning; Fountain Syndrome; Fournier Gangrene; Fox Fordyce Disease; Fragile X Syndrome; Fraser Syndrome; Freeman Sheldon Syndrome; Frey's Syndrome; Froelich's Syndrome; Frontofacionasal Dysplasia; Frontonasal Dysplasia; Fructose Intolerance, Hereditary; Fructosuria; Fryns Syndrome; ; Fukuyama Type Congenital Muscular Dystrophy; Galactosemia; Galloway Mowat Syndrome; Gardner Syndrome; Gastritis, Chronic, Erosive; Gastritis, Giant Hypertrophie; Gastroenteritis, Eosinophilic; Gastroesophageal Reflux; Gastrointestinal Stromal Tumors; Gastroschisis; Gaucher Disease; Gerstmann Syndrome; Gianotti Crosti Syndrome; Giant Cell Myocarditis; Giardiasis; Gilbert Syndrome; Glanzmann Thrombasthenia; Glioblastoma Multiforme; Glucose Galactose Malabsorption; Glucose-6-Phosphate Dehydrogenase Deficiency; Glutaricaciduria I; Glutaricaciduria II; Glycogen Storage Disease Type V; Glycogen Storage Disease VIII; Goldenhar Syndrome (Oculo Auriculo Vertebral Spectrum); Goodman Syndrome; Goodpasture Syndrome; Gordon Syndrome; Gorham's Disease; Gorlin Chaudhry Moss Syndrome; Gottron Syndrome; Graft versus Host Disease; Granuloma Annulare; Granulomatosis, Lymphomatoid; Granulomatous Disease, Chronic; Graves' Disease; Greig Cephalopolysyndactyly Syndrome; Grover's Disease; Growth Delay, Constitutional; Growth Hormone Deficiency; Guillain Barre Syndrome; Hageman Factor Deficiency; Hajdu Cheney Syndrome; Hallermann Streiff syndrome; Hand Foot Mouth Syndrome; Hanhart Syndrome; Hantavirus Pulmonary Syndrome; Hartnup Disease; Hay-Wells Syndrome; Headache, Cluster; Heart Block, Congenital; Heavy Metal Poisoning; Hemangioma Thrombocytopenia Syndrome; Hematuria, Benign, Familial; Hemochromatosis, Hereditary; Hemoglobinuria, Paroxysmal Cold; Hemoglobinuria, Paroxysmal Nocturnal; Hemolytic Uremic Syndrome; Hemophilia; Hemorrhagic Telangiectasia, Hereditary; Hepatic Fibrosis, Congenital; Hepatitis B; Hepatitis C; Hepatitis, Neonatal; Hepatorenal Syndrome; Hermansky Pudlak Syndrome; Hermaphroditism, True; Herpes, Neonatal; Hers Disease; Hiccups, Chronic; Hidradenitis Suppurativa; Hirschsprung's Disease; Histidinemia; Hodgkin's Disease; Holoprosencephaly; Holt Oram Syndrome; Homocystinuria; Horner's Syndrome; Human Granulocytic Ehrlichiosis (HGE); Human Monocytic Ehrlichiosis (HME); Hunter Syndrome; Huntington's Disease; Hydranencephaly; Hydrocephalus; Hyper IgM Syndrome; Hypercholesterolemia; Hyperemesis Gravidarum; Hyperexplexia; Hyperhidrosis, Primary; Hyperkalemia; Hyperlipoproteinemia Type III; Hyperlipoproteinemia Type IV; Hyperostosis Frontalis Interna; Hyperoxaluria, Primary (Type I); Hyperprolinemia Type I; Hyperprolinemia Type II; Hyperthermia; Hypochondroplasia; Hypoglycemia; Hypohidrotic Ectodermal Dysplasia; Hypokalemia; Hypomelanosis of Ito; Hypoparathyroidism; Hypophosphatasia; Hypophosphatemia, Familial; Hypoplastic Left Heart Syndrome; Hypotension, Orthostatic; Hypothyroidism; Hypotonia, Benign Congenital; I Cell Disease; IRF6-Related Disorders; Ichthyosis; Ichthyosis Hystrix, Curth Macklin Type; Ichthyosis Vulgaris; Ichthyosis, CHILD Syndrome; Ichthyosis, Chanarin Dorfman Syndrome; Ichthyosis, Erythrokeratodermia Progressiva Symmetrica; Ichthyosis, Erythrokeratodermia Variabilis; Ichthyosis, Erythrokeratolysis Hiemalis; Ichthyosis, Harlequin Type; Ichthyosis, Keratosis Follicularis Spinulosa Decalvans; Ichthyosis, Lamellar; Ichthyosis, Netherton Syndrome; Ichthyosis, Sjogren Larsson Syndrome; Ichthyosis, Trichothiodystrophy; Ichthyosis, X Linked; Idiopathic Pulmonary Fibrosis; IgA Nephropathy; Imperforate Anus; Incontinentia Pigmenti; Interstitial Cystitis; Intestinal Pseudoobstruction; Irritable Bowel Syndrome; Ivemark Syndrome; Jackson Weiss Syndrome; Jansen Type Metaphyseal Chondrodysplasia; Jarcho Levin Syndrome; Jejunal Atresia; Jervell and Lange-Nielsen Syndrome; Job Syndrome; Johanson Blizzard Syndrome; Joubert Syndrome; Jumping Frenchmen of Maine; KBG Syndrome; Kabuki Make-up Syndrome; Kallmann Syndrome; Kartagener Syndrome; Kawasaki Disease; Kearns Sayre Syndrome; Kennedy Disease; Kenny Caffey Syndrome; Keratitis Ichthyosis Deafness Syndrome; Keratoconjunctivitis, Vernal; Keratoconus; Keratomalacia; Keratosis Follicularis; Keratosis, Seborrheic; Kernicterus; Kienbock Disease; Kikuchi's Disease; Kleine Levin Syndrome; Klinefelter Syndrome; Klippel Trenaunay Syndrome; Klippel-Feil Syndrome; Kluver Bucy Syndrome; Kniest Dysplasia; Kohler Disease; Kufs Disease; Kugelberg Welander Syndrome; L1 Syndrome; LADD Syndrome; LEOPARD Syndrome; Laband Syndrome; Lactose Intolerance; Lambert-Eaton Myasthenic Syndrome; Landau Kleffner Syndrome; Langerhans Cell Histiocytosis; Laron Syndrome; Larsen Syndrome; Laurence Moon Syndrome; Leber Hereditary Optic Neuropathy; Leber's Congenital Amaurosis; Legg Calve Perthes Disease; Legionnaires' Disease; Leigh's Disease; Lennox Gastaut Syndrome; Lenz Microphthalmia Syndrome; Leprechaunism; Leprosy; Leptospirosis; Leri Pleonosteosis; Lesch Nyhan Syndrome; Leukemia, Chronic Lymphocytic; Leukemia, Chronic Myelogenous; Leukemia, Hairy Cell; Leukodystrophy; Leukodystrophy, Krabbe's; Leukodystrophy, Metachromatic; Lichen Planus; Lichen Sclerosus; Lipodystrophy; Lissencephaly; Listeriosis; Locked In Syndrome; Loken Senior Syndrome; Lowe Syndrome; Lupus; Lyme Disease; Lymphadenopathy, Angioimmunoblastic with Dysproteinemia; Lymphangioleiomyomatosis; Lymphatic Malformations; Lymphedema, Hereditary; Lymphocytic Infiltrate of Jessner; Lymphoma, Gastric, Non Hodgkins Type; Lynch Syndromes; Lysosomal Storage Disorders; MELAS Syndrome; MERRF Syndrome; MURCS Association; Machado-Joseph Disease; Macroglossia; Macular Degeneration; Madelung's Disease; Maffucci Syndrome; Mal de Debarquement; Malaria; Malignant Hyperthermia; Mallory Weiss Syndrome; Manic Depression, Bipolar; Mantle Cell Lymphoma; Maple Syrup Urine Disease; Marcus Gunn Phenomenon; Marden Walker Syndrome; Marfan Syndrome; Marinesco Sjogren Syndrome; Maroteaux Lamy Syndrome; Marshall Smith Syndrome; Marshall Syndrome; Mastocytosis; Maxillofacial Dysostosis; Maxillonasal Dysplasia, Binder Type; May Hegglin Anomaly; McCune Albright Syndrome; McKusick Type Metaphyseal Chondrodysplasia; Measles; Meckel Syndrome; Mediterranean Fever, Familial; Medium Chain Acyl CoA Dehydrogenase Deficiency; Medullary Cystic Kidney Disease/Nephronophthisis; Medullary Sponge Kidney; Medulloblastoma; Megalocornea Mental Retardation Syndrome; Meige Syndrome; Melanoma, Malignant; Meleda Disease; Melkersson Rosenthal Syndrome; Melnick Needles Syndrome; Membranoproliferative Glomerulonephritis Type II; Meniere Disease; Meningioma; Meningitis; Meningitis, Bacterial; Meningitis, Meningococcal; Meningitis, Tuberculous; Meningococcemia; Menkes Disease; Mesenteritis, Retractile; Mesothelioma; Metaphyseal Chondrodysplasia, Schmid Type; Metatropic Dysplasia I; Microvillus Inclusion Disease; Mikulicz Syndrome; Miller Syndrome; Mitral Valve Prolapse Syndrome; Mixed Connective Tissue Disease (MCTD); Moebius Syndrome; Monilethrix; Morquio Syndrome; Motor Neuron Disease; Mountain Sickness, Acute; Mowat-Wilson Syndrome; Moyamoya Syndrome; Mucha Habermann Disease; Mucolipidosis IV; Mucopolysaccharidoses; Mucopolysaccharidosis Type I; Mucopolysaccharidosis Type III; Mucous Membrane Pemphigoid; Mulibrey Nanism Syndrome (Perheentupa Syndrome); Mullerian Aplasia; Multiple Epiphyseal Dysplasia; Multiple Sclerosis; Multiple Sulfatase Deficiency; Multiple System Atrophy; Mulvihill Smith Syndrome; Mumps; Muscular Dystrophy, Becker; Muscular Dystrophy, Duchenne; Muscular Dystrophy, Emery Dreifuss; Muscular Dystrophy, Limb Girdle; Muscular Dystrophy, Oculo Gastrointestinal; Mutism, Selective; Myasthenia Gravis; Mycosis Fungoides; Myelodysplastic Syndromes; Myelofibrosis, Idiopathic; Myeloma, Multiple; Myhre Syndrome; Myoclonus, General; Myopathy, Congenital, Batten Turner Type; Myopathy, Desmin Storage; Myopathy, Scapuloperoneal; Myositis, Inclusion Body; Myotonia Congenita; Myotubular Myopathy; N-Acetyl Glutamate Synthetase Deficiency; Nager Syndrome; Nail Patella Syndrome; Narcolepsy; Nelson Syndrome; Nemaline Myopathy; Neonatal Lupus; Neu Laxova Syndrome; Neurasthenia; Neuroacanthocytosis; Neurodegeneration with Brain Iron Accumulation Type 1; Neurofibromatosis Type 1 (NF-1); Neurofibromatosis Type 2 (NF-2); Neuroleptic Malignant Syndrome; Neuromyotonia; Neuropathy, Ataxia and Retinitis Pigmentosa; Neuropathy, Congenital Hypomyelination; Neuropathy, Giant Axonal; Neuropathy, Hereditary Sensory, Type I; Neuropathy, Hereditary Sensory, Type II; Neuropathy, Hereditary Sensory, Type IV; Neuropathy, Peripheral; Neutropenia, Cyclic; Neutropenia, Severe Chronic; Nevoid Basal Cell Carcinoma Syndrome; Nezelofs Syndrome; Niemann Pick Disease; Nocardiosis; Nonketotic Hyperglycinemia; Noonan Syndrome; Norrie Disease; Nystagmus, Benign Paroxysmal Positional;Obsessive Compulsive Disorder; Ochoa Syndrome; Ocular Motor Apraxia, Cogan Type; Oculo-Dento-Digital Dysplasia; Oculocerebral Syndrome with Hypopigmentation; Oculocerebrocutaneous Syndrome; Olivopontocerebellar Atrophy, Hereditary; Ollier Disease; Opitz G/BBB Syndrome; Opportunistic Infections; Opsoclonus-Myoclonus Syndrome; Oral Facial Digital Syndrome; Organic Personality Syndrome; Ornithine Transcarbamylase Deficiency; Orocraniodigital Syndrome; Osgood Schlatter Condition; Osteogenesis Imperfecta; Osteomyelitis; Osteonecrosis; Osteopetrosis; Otopalatodigital Syndrome Type I and II; PEPCK Deficiency, Mitochondrial; POEMS Syndrome; Pachydermoperiostosis; Pachyonychia Congenita; Paget's Disease; Paget's Disease of the Breast; Pallister Hall Syndrome; Pallister Killian Mosaic Syndrome; Pallister W Syndrome; Pancreatic Islet Cell Tumor; Panic Anxiety Syndrome; Panniculitis, Idiopathic Nodular; Papillitis; Papillon Lefevre Syndrome; Paracoccidioidomycosis; Paramyotonia Congenita; Paraplegia, Hereditary Spastic; Parkinson's Disease; Parry Romberg Syndrome; Pars Planitis; Parsonage Turner Syndrome; Patulous Eustachian Tube; Pediatric Cardiomyopathy; Peeling Skin Syndrome; Pelizaeus Merzbacher Brain Sclerosis; Pemphigus; Penta X Syndrome; Pentalogy of Cantrell; Perisylvian Syndrome, Congenital Bilateral; Perniosis; Pertussis; Peutz Jeghers Syndrome; Peyronie Disease; Pfeiffer Syndrome Type I; Phelan-McDermid Syndrome; Phenylketonuria; Pheochromocytoma; Phocomelia Syndrome; Phosphoglycerate Kinase Deficiency; Pica; Pick's Disease; Pierre Robin Sequence; Pineal Cysts, Symptomatic; Pinta; Pityriasis Rubra Pilaris; Pleuropulmonary Blastoma; Pneumonia, Eosinophilic; Pneumonia, Interstitial; Poland Syndrome; Polyarteritis Nodosa; Polychondritis; Polycystic Kidney Diseases; Polycystic Liver Disease; Polycystic Ovary Syndrome; Polycythemia Vera; Polyglucosan Body Disease, Adult; Polymorphous Low-Grade Adenocarcinoma; Polymyalgia Rheumatica; Polymyositis; Pompe Disease; Porphyria; Porphyria Cutanea Tarda; Porphyria, ALA-D; Porphyria, Acute Intermittent; Porphyria, Congenital Erythropoietic; Porphyria, Hereditary Coproporphyria; Porphyria, Variegate; Post Polio Syndrome; Posterior Uveitis; Prader Willi Syndrome; Precocious Puberty; Primary Lateral Sclerosis; Primary Orthostatic Tremor; Proctitis; Progeria, Hutchinson Gilford; Progressive Myoclonus Epilepsy; Progressive Osseous Heteroplasia (POH); Progressive Supranuclear Palsy; Prostatitis; Proteus Syndrome; Prune Belly Syndrome; Pseudo Hurler Polydystrophy; Pseudoachondroplastic Dysplasia; Pseudocholinesterase Deficiency; Pseudohypoparathyroidism; Pseudomyxoma Peritonei; Pseudotumor Cerebri; Pseudoxanthoma Elasticum (PXE); Psittacosis; Psoriasis; Pterygium Syndrome, Multiple; Pulmonary Alveolar Proteinosis; Pulmonary Hypertension, Primary; Pulmonary Hypertension, Secondary; Pure Red Cell Aplasia, Acquired; Purpura, Henoch-Schonlein; Purpura, Idiopathic Thrombocytopenic; Purpura, Thrombotic Thrombocytopenic; Pyknodysostosis; Pyoderma Gangrenosum; Pyridoxine-Dependent Seizures; Pyruvate Carboxylase Deficiency; Pyruvate Dehydrogenase Deficiency; Pyruvate Kinase Deficiency; Q Fever; Rabies; Rabson-Mendenhall Syndrome; Radiation Syndromes; Ramsay-Hunt Syndrome; Rapp Hodgkin Syndrome; Raynaud's Disease and Phenomenon; Recurrent Respiratory Papillomatosis; Reflex Sympathetic Dystrophy Syndrome; Refsum Disease; Reiter's Syndrome; Renal Agenesis, Bilateral; Renal Glycosuria; Respiratory Distress Syndrome, Infant; Restless Legs Syndrome; Retinitis Pigmentosa; Retinoblastoma; Retinopathy of Prematurity; Retinopathy, Arteriosclerotic; Retinopathy, Diabetic; Retinopathy, Hypertensive; Retinoschisis; Retroperitoneal Fibrosis; Rett Syndrome; Reye Syndrome; Rh Disease; Rheumatic Fever; Rickets, Vitamin D Deficiency; Rieger Syndrome; Roberts Syndrome; Robinow Syndrome; Rocky Mountain Spotted Fever; Romano Ward Syndrome; Rosai-Dorfman Disease; Rosenberg Chutorian Syndrome; Roseola Infantum; Rothmund Thomson Syndrome; Roussy Levy Syndrome; Rubella; Rubella, Congenital; Rubinstein Taybi Syndrome; Russell Silver Syndrome (RSS); Ruvalcaba Syndrome; SHORT Syndrome; Saethre Chotzen Syndrome; Sakati Syndrome; Sandhoff Disease; Santavuori Disease; Sarcoidosis; Schindler Disease; Schinzel Giedion Syndrome; Schinzel Syndrome; Schwartz Jampel Syndrome; Scleroderma; Scott Craniodigital Syndrome; Seckel Syndrome; Seitelberger Disease (Infantile Neuroaxonal Dystrophy); Sennetsu Fever; Septooptic Dysplasia; Setleis Syndrome; Severe Combined Immunodeficiency; Sheehan Syndrome; Short Chain Acyl CoA Dehydrogenase Deficiency (SCAD); Shwachman Syndrome; Sialadenitis; Sialidosis; Sickle Cell Disease; Simian B Virus Infection; Simpson Dysmorphia Syndrome; Singleton Merten Syndrome; Sinonasal Undifferentiated Carcinoma; Sirenomelia Sequence; Sjogren Syndrome; Sly Syndrome; Smallpox; Smith Lemli Opitz Syndrome; Smith Magenis Syndrome; Sneddon Syndrome; Sotos Syndrome; Spasmodic Dysphonia; Spasmodic Torticollis; Spina Bifida; Spinal Muscular Atrophy; Split Hand/Split Foot Malformation; Spondyloepiphyseal Dysplasia Tarda; Spondyloepiphyseal Dysplasia, Congenital; Sprengel Deformity; Stenosis, Spinal; Stevens Johnson Syndrome; Stickler Syndrome; Stiff Person Syndrome; Streptococcus, Group B; Sturge Weber Syndrome; Stuve-Wiedemann Syndrome; Subacute Sclerosing Panencephalitis; Succinic Semialdehyde Dehydrogenase Deficiency; Sudden Infant Death Syndrome; Summitt Syndrome; Susac Syndrome; Sutton Disease II; Sweet Syndrome; Syphilis, Acquired; Syphilis, Congenital; Syringobulbia; Syringomyelia; TORCH Syndrome; Tangier Disease; Tardive Dyskinesia ; Tarsal Tunnel Syndrome; Tarui Disease; Tay Sachs Disease; Telecanthus with Associated Abnormalities; Temporomandibular Joint Dysfunction (TMJ); Tethered Spinal Cord Syndrome; Tetrahydrobiopterin Deficiency; Tetralogy of Fallot; Thalamic Syndrome (Dejerine Roussy); Thalassemia Major; Thalassemia Minor; Three M Syndrome; Thrombocythemia, Essential; Thrombocytopenia Absent Radius Syndrome; Thrombocytopenia, Essential; Tietze Syndrome; Timothy Syndrome; Tinnitus; Tolosa Hunt Syndrome; Tongue Carcinoma; Tongue, Fissured; Tongue, Geographie; Tongue, Hairy; Tooth and Nail Syndrome; Tourette Syndrome; Townes Brocks Syndrome; Toxic Epidermal Necrolysis; Toxic Shock Syndrome; Toxocariasis; Toxoplasmosis; Transverse Myelitis; Treacher Collins Syndrome; Tricho Dento Osseous Syndrome; Trichorhinophalangeal Syndrome Type I; Trichorhinophalangeal Syndrome Type III; Trichotillomania; Trigeminal Neuralgia (Tic Douloureux); Trimethylaminuria; Triplo X Syndrome; Triploid Syndrome; Trismus Pseudocamptodactyly Syndrome; Trisomy; Trisomy 13 Syndrome; Trisomy 18 Syndrome; Tropical Sprue; Truncus Arteriosus; Tuberculosis; Tuberous Sclerosis; Tularemia; Turcot Syndrome; Turner Syndrome; Twin Twin Transfusion Syndrome; Typhoid; Tyrosinemia, Hereditary; Urticaria Pigmentosa; Urticaria, Cholinergic; Urticaria, Cold; Urticaria, Papular; Urticaria, Physical; Usher Syndrome; VACTERL Association; VACTERL with Hydrocephalus; Valinemia; Varicella Zoster; Vascular Malformations of the Brain; Vasculitis; Vasculitis, Cutaneous Necrotizing; Velocardiofacial Syndrome; Ventricular Septal Defects; Very Long Chain Acyl CoA Dehydrogenase Deficiency (LCAD); Vitamin B12 Deficiency; Vitiligo; Vogt Koyanagi Harada Syndrome; Von Gierke Disease; Von Hippel Lindau Disease; Von Willebrand Disease; Vulvovaginitis; WAGR Syndrome; Waardenburg Syndrome; Waldenstrom's Macroglobulinemia; Waldmann Disease; Walker Warburg Syndrome; Wandering Spleen; Weaver Syndrome; Wegener's Granulomatosis; Weil Syndrome; Weill Marchesani Syndrome; Weismann Netter Stuhl Syndrome; Werdnig Hoffman Disease; Werner Syndrome; Wernicke-Korsakoff Syndrome; West Nile Encephalitis; West Syndrome; Whipple Disease; Wieacker Syndrome; Wiedemann Rautenstrauch Syndrome; Wildervanck Syndrome; Williams Syndrome; Wilms' Tumor; Wilson's Disease; Winchester Syndrome; Wiskott Aldrich Syndrome; Wolf Hirschhorn Syndrome; Wolff Parkinson White Syndrome; Wolfram Syndrome; Wyburn Mason Syndrome; X linked Juvenile Retinoschisis; X linked Lymphoproliferative Syndrome; XYY Syndrome; Xeroderma Pigmentosum; Yaws; Yellow Fever; Yellow Nail Syndrome; Yunis Varon Syndrome; Zellweger Syndrome; Zollinger Ellison Syndrome.

Wobei die Emulsionen wie folgt definiert sind:
Bei der erfindungsgemäßen Zubereitungsform handelt es sich um eine Öl-in-Wasser Zubereitung, bei welcher der Öl-Anteil bis zu 70 % (m/m) betragen kann, vorzugsweise bis zu 50% und noch bevorzugter bis zu 30%. Ferner gemäß einer weiteren bevorzugten Ausführungsform besteht der Öl-Anteil aus einer, zwei, drei, oder vier verschiedenen Ölen. Alternativ können auch fünf oder mehr verschiedene Öle miteinander den Öl-Anteil bilden.

Die eingesetzten Öle umfassen Sojaöl, Olivenöl, Omega-3 triglyceride (z. B. Fischöl), mittelkettige Triglyceride. Die Emulgatoren (Eilecithin, Sojalecithin) entsprechen dem Stand der Technik für parenterale Zubereitungen. Ferner können als Öle eingesetzt werden: natürlich Öle, synthetische Öle, Aloeöl, Baumwollsamenöl, Bombyx-Mori-Öl, Borretschöl, Cajuputöl, Carthamusöl, Castor-Öl, Chrysalisöl, Eiöl, Fischöl, Fischlebertranöl, Haileberöl, Hanfsamenöl, Harzöl, Haselnussöl, Hautfette, Knochenfette, Heringsöl, Himbeerkernöl, Holzöl, Jojobaöl, Kabeljauöl, Kamelienöl, Kapoköl, Kokosnussöl (Kokosöl), Leinöl, Lorbeeröl, Maisöl, Mandelöl, Mohnöl, Neemöl (Margosaöl oder Nimöl), Nigersamenöl, Obstkernöl, Oiticicaöl, Olivenöl, Oliventresteröl, Onagraöl, Palmöl, Palmkernöl, Pilchardöl (Kanadasardinenöl), Pinienöl, Rapsöl, Rizinusöl, Safloröl, Samenöl von Gummibäumen, Schibutter (Karite-Butter), Sesamöl, Sisymbrinöl, Sojaöl, Sojabohnenöl, Sonnenblumenöl, Traubenkernöl, Weizenkeimöl.

In einem weiteren Aspekt bezieht sich die beanspruchte Erfindung auf ein Verfahren zur Herstellung einer Öl-in-Wasser-Emulsion, wie vorstehend beschrieben, umfassend wenigstens die folgenden Schritte:
(A) Inertisieren wenigstens einer Ölphase und wenigstens einer wässrigen Phase, wobei wenigstens ein Cannabinoid der wenigstens einen Ölphase zugesetzt wird,
(B) Erwärmen der wenigstens einen Ölphase auf eine Temperatur im Bereich von ≥ 20 °C bis ≤ 70 °C und erwärmen der wenigstens einen wässrigen Phase auf eine Temperatur im Bereich von ≥ 20 °C bis ≤ 70 °C,
(C) Mischen der wenigstens einen Ölphase und der wenigstens einen wässrigen Phase um eine Präemulsion zu erhalten,
(D) Homogenisieren der Präemulsion, um die Öl-in-Wasser-Emulsion zu erhalten.

In einer bevorzugen Ausführungsform umfasst in Schritt (A) das Inertisieren der wenigstens einen Ölphase und der wenigstens einen wässrigen Phase das Einblasen von Stickstoff mittels wenigstens einer Sonde und kontinuierliches Abziehen von Sauerstoff umfasst. Bevor die Feststoffe, wie das wenigstens eine Cannabinoid und der wenigstens eine Emulgator, in die Ölphase bzw. wässrige Phase eingebracht werden, müssen diese weitestgehend vom Sauerstoff befreit werden. Dieser Prozess wird als Inertisierung bezeichnet. Hierzu wird Stickstoff mittels Sonde eingeblasen und wieder abgezogen. Dabei wird der Sauerstoffgehalt kontinuierlich durch physikalische Gleichgewichtseinstellung gesenkt. Der Sauerstoffgehalt in der Flüssigkeit und im Gasraum wird mittels Sauerstoffsonden kontrolliert. Zum Schluss werden die Feststoffe zugeführt und die Flüssigkeiten weiter begast, um sicherzustellen, dass die erfindungsgemäß beanspruchte Öl-in-Wasser-Emulsion unter optimalen Bedingungen hergestellt wird und die Stabilität des wenigstens einen Cannabinoids gewährleistet ist. In einer bevorzugten Ausführungsform wird der Sauerstoffgehalt der wenigstens einen Ölphase auf einen Sauerstoffgehalt von ≤ 3,5 mg/L und der wenigstens einen wässrigen Phase in Schritt (A) jeweils auf einen Sauerstoffgehalt von ≤ 0,5 mg/L, bezogen auf die wenigstens eine Ölphase oder wenigstens eine wässrige Phase, reduziert.

In Abhängigkeit von der Löslichkeit der Feststoffe werden die wässrige Phase und/oder die Ölphase in Schritt (B) auf eine Temperatur zwischen 20 °C und 70 °C erwärmt. Dieser Schritt erfolgt, bevor die Phasen in Schritt (C) weiterverarbeitet werden. Durch die Erwärmung wird sichergestellt, dass die Feststoffe vollständig in der jeweiligen Phase gelöst werden, wodurch eine homogene Mischung und optimale Bedingungen für die anschließende Verarbeitung geschaffen werden.

In einer bevorzugten Ausführungsform umfasst der Schritt (C) das Mischen der wenigstens einen Ölphase und der wenigstens einen wässrigen Phase unter Verwendung wenigstens eines Rotor-Stator-Dispergators. Ein Rotor-Stator-Dispergator ist ein Dispergiergerät, das nach dem Rotor-Stator-Prinzip arbeitet und intensive Scherkräfte erzeugt, um Tröpfchen zu zerkleinern und Emulsionen zu homogenisieren. Diese Geräte bestehen aus einem rotierenden Rotor und einem stationären Stator, die zusammen eine Scherzone bilden, in der die Flüssigkeiten stark beschleunigt und vermischt werden. In einer Ausgestaltung können Rotor-Stator-Dispergatoren beispielsweise Scherenschlitze aufweisen, deren Breite, Anzahl und gegenseitiger Abstand die Scherwirkung und den spezifischen Energieeintrag beeinflussen. Die Zuführung der Ölphase und der wässrigen Phase erfolgt getrennt, was oft durch ein Koaxialrohr oder einen Koaxialschlauch realisiert wird. Dabei wird die Ölphase durch das innere Rohr und die wässrige Phase durch das äußere Rohr geführt, um eine effektive und gleichmäßige Durchmischung zu gewährleisten. Eine Anlage zur Herstellung der erfindungsgemäß beanspruchten Öl-in-Wasser-Emulsion kann eine Mehrzahl von Rotor-Stator-Dispergatoren umfassen, die entweder parallel oder in Reihe geschaltet sind. Durch die Parallel- und/oder Reihenschaltung lässt sich sowohl die Prozessproduktivität als auch die Prozessqualität erhöhen, ohne die Prozesszeit zu verlängern.

In einer bevorzugten Ausführungsform werden die wenigstens eine Ölphase und die wenigstens eine wässrige Phase mittels einer Rohr-in-Rohr-Anordnung und/oder mittels wenigstens einem statischen Mischer dem wenigstens einen Rotor-Stator-Dispergator zugeführt. In einer bevorzugten Ausführungsform werden die englische Methode und/oder die kontinentale Methode zur Herstellung der erfindungsgemäß beanspruchten Öl-in-Wasser-Emulsionen verwendet.

In einer bevorzugten Ausführungsform werden die wenigstens eine Ölphase und die wenigstens eine wässrige Phase durch eine Scherzone dem wenigstens einen Rotor-Stator-Dispergator zugeführt. Die Scherzone eines Rotor-Stator-Dispergators ist der Bereich, in dem die intensive Scherung und Zerkleinerung der Tropfen erfolgt. Innerhalb dieser Zone bewirken die rotierenden und stationären Komponenten, dass Flüssigkeiten mit hoher Geschwindigkeit und unter hohen Scherkräften vermischt werden, was zur Zerkleinerung der Tröpfchen führt. Diese Zone ist entscheidend für die Homogenisierung und Emulgierung der Phasen. Die Scherzone kann spezifische Merkmale wie Scherenschlitze aufweisen, deren Breite, Anzahl und gegenseitiger Abstand die Effizienz der Scherung beeinflussen. Für die Zuführung der Phasen kann der Rotor-Stator-Dispergator mit einem Koaxialrohr oder Koaxialschlauch ausgestattet sein, wobei die Ölphase durch das innere Rohr und die wässrige Phase durch das äußere Rohr geführt wird. Dies gewährleistet eine präzise und getrennte Zuführung der Phasen in die Scherzone. In einer bevorzugten Ausführungsform erfolgt in Schritt (C) das Mischen der wenigstens einen Ölphase und der wenigstens einen wässrigen Phase bei einer Drehzahl im Bereich von ≥ 4.000 U/min bis ≤ 16.000 U/min. In einer bevorzugten Ausführungsform weist die Präemulsion eine mittlere Teilchengröße im Bereich von ≥ 0,5 bis ≤ 500 µm auf.

In einer bevorzugten Ausführungsform erfolgt in Schritt (D) das Homogenisieren der Präemulsion unter Verwendung wenigstens eines Hochdruckhomogenisators.

Besonders bevorzugt ist der wenigstens eine Hochdruckhomogenisator ein Kolben-Spalt-Homogenisator. Ein Kolben-Spalt-Homogenisator ist ein Gerät, das zur Erzeugung sehr feiner Emulsionen und Suspensionen verwendet wird. Es arbeitet, indem es eine Flüssigkeit wie beispielsweise die Präemulsion mit hohem Druck durch einen engen Spalt oder eine Düse presst. Dabei entstehen extreme Scherkräfte, Turbulenzen und Kavitation, die die dispergierten Partikel oder Tröpfchen aufbrechen und auf eine sehr kleine Größe reduzieren.

Bevorzugt wird die Präemulsion in Schritt (D) 1, 2, 3, 4, 5, 6, 7, 8, 9 oder 10 Zyklen, bevorzugt in 2, 3, 4 oder 5 Zyklen, der Homogenisation im Kolben-Spalt-Homogenisator unterworfen, um die gewünschte Tropfengröße zu erreichen. Bevorzugt wird die Homogenisation in Schritt (D) im Kolben-Spalt-Homogenisator bei einem Druck im Bereich von ≥ 300 bar bis ≤ 800 bar durchgeführt.

Besonders bevorzugt ist der wenigstens eine Hochdruckhomogenisator ein Gegenstrahldispergator. Ein "Gegenstrahldispergator" ist ein Hochdruckhomogenisator, bei dem zwei oder mehr Strahlen einer Präemulsion aus mindestens zwei, vorzugsweise zwei gegenüberliegenden, Bohrungen oder Kanälen in einer Tropfenzerkleinerungszone aufeinandertreffen. Durch das Aufeinandertreffen der Präemulsionsstrahlen findet insbesondere unter der Einwirkung von Scherkräften eine Tropfenzerkleinerung der in der Präemulsion enthaltenen Tropfen statt. Das Ausmaß der Tropfenzerkleinerung hängt von der Fördergeschwindigkeit ab, mit der die Öl-in-Wasser-Präemulsion oder deren Strahlen innerhalb des Gegenstrahldispergators befördert werden.

In einer bevorzugten Ausführungsform, weist ein Gegenstrahldispergator vorzugsweise wenigstens zwei, insbesondere zwei gegenüberliegende Kanäle auf, die eine Y-förmige Konfiguration oder Anordnung haben. Diese Kanäle besitzen typischerweise einen Innendurchmesser im Mikrometerbereich, wodurch eine besonders intensive Scherung der in der Öl-in-Wasser-Präemulsion enthaltenen Tropfen erzielt werden kann. Diese Konstruktion ermöglicht eine effektive Tropfenzerkleinerung und damit die Herstellung von Öl-in-Wasser-Emulsionen mit einer engen oder schmalen Tröpfchengrößenverteilung.

Bevorzugt werden in Schritt (D) mehrere Gegenstrahldispergatoren hintereinandergeschaltet. Bevorzugt erfolgt die Homogenisation in Schritt (D) in einem Gegenstrahldispergator bei einem Pumpendruck im Bereich von ≥ 500 bar bis ≤ 2000 bar.

Der Pumpendruck bezeichnet den von einer Pumpe, insbesondere einer Hochdruckpumpe, erzeugten Druck, der für die Förderung und Aufprallgeschwindigkeit von Emulsionsstrahlen innerhalb eines Gegenstrahldispergators verantwortlich ist. In einem Gegenstrahldispergator treffen zwei oder mehr Strahlen einer Präemulsion aus mindestens zwei gegenüberliegenden Kanälen in einer Tropfenzerkleinerungszone aufeinander. Der Pumpendruck ist entscheidend für die Steuerung der Scherkräfte, die die Tröpfchenzerkleinerung und damit die Homogenisierung der Emulsion bewirken. Typischerweise wird der Pumpendruck im Bereich von ≥ 500 bis ≤ 2000 bar eingestellt. Beispielsweise kann ein erster Gegenstrahldispergator bei einem höheren Pumpendruck von etwa 1500 bar betrieben werden, während ein nachgeschalteter Gegenstrahldispergator bei einem niedrigeren Druck von etwa 500 bar arbeitet. Diese Druckkaskade ermöglicht die Herstellung von Emulsionen mit einer engen Tropfengrößenverteilung und einer signifikanten Reduktion des Anteils an größeren Tröpfchen. Kommerziell erhältliche Geräte wie die PSI-Serie^{®} oder Microfluidizer^{®} verwenden solche Hochdruckhomogenisatoren zur effizienten Emulgierung.

Bevorzugt wird die Präemulsion in Schritt (D) bei einer Temperatur im Bereich von ≥ 20 °C bis ≤ 80 °C, besonders bevorzugt bei einer Temperatur im Bereich von ≥ 30 °C bis ≤ 75 °C, homogenisiert. Ein Gegenstrahldispergator kann zum Durchführen von Schritt (C) bei verschiedenen Pumpendrücken und Temperaturen betrieben werden, um die Homogenisierung der Öl-in-Wasser-Emulsion zu optimieren. Beispielsweise kann der Gegenstrahldispergator bei einem Pumpendruck von 1500 bar und einer Temperatur der Präemulsion von 50 °C betrieben werden. Weiterhin kann der Gegenstrahldispergator zum Durchführen von Schritt (C) bei einem Pumpendruck von 1900 bar und einer Temperatur der Präemulsion von 40 °C oder bei einem Pumpendruck von 1000 bar und einer Temperatur der Präemulsion von 60 °C betrieben werden. Diese Parameter ermöglichen eine effektive Tropfenzerkleinerung und die Herstellung einer stabilen Emulsion mit einer engen Tropfengrößenverteilung.

In einer bevorzugten Ausführungsform wird die Präemulsion beim Durchführen von Schritt (C) mehrmals, insbesondere zwei Mal, drei Mal, vier Mal oder fünf Mal, durch den wenigstens einen Gegenstrahldispergator geleitet. Durch ein mehrmaliges Durchleiten der Präemulsion durch den wenigstens einen Gegenstrahldispergator ist mit besonderem Vorteil eine Erhöhung der Prozessqualität, insbesondere in Bezug auf den mittleren Tropfendurchmesser und/oder den pFAT₅-Wert, erzielbar. In einer weiteren bevorzugten Ausführungsform wird der Schritt (C) mittels einer Mehrzahl von Gegenstrahldispergatoren, insbesondere mittels zwei, drei, vier oder fünf Gegenstrahldispergatoren, durchgeführt. Bevorzugt wird der Schritt (C) mittels einer Mehrzahl von parallel geschalteten Gegenstrahldispergatoren und/oder mittels einer Mehrzahl von in Reihe geschalteten Gegenstrahldispergatoren durchgeführt. Durch die Verwendung einer Mehrzahl von Gegenstrahldispergatoren, insbesondere durch eine Parallel- und/oder Reihenschaltung der Gegenstrahldispergatoren, ist ebenfalls eine signifikante Verbesserung der Prozessqualität, insbesondere in Bezug auf den mittleren Tropfendurchmesser und/oder den pFAT₅-Wert, erzielbar. Zusätzlich lässt sich durch diese Verfahrensmaßnahme(n) die Prozessproduktivität erhöhen. So verändert sich die Prozesszeit im Falle einer Reihenschaltung der Gegenstrahldispergatoren nicht. Im Falle einer Parallelschaltung verringert sich die Prozesszeit linear. Insgesamt ergibt sich daher eine beträchtliche Zeitersparnis, wodurch eine signifikante Erhöhung der Anzahl an produzierbaren Emulsionschargen pro Zeiteinheit und mithin eine signifikante Steigerung der Prozessproduktivität erzielbar ist.

In einer bevorzugten Ausführungsform umfasst das erfindungsgemäß beanspruchte Verfahren den Schritt (E): Abfüllen der Öl-in-Wasser-Emulsion unter Schutzgas in Primärpackmittel und Sterilisation in einem Autoklaven. Das Primärpackmittel wird bevorzugt ausgewählt aus der Gruppe bestehend aus Glasvials, Ampullen, Injektionsbeuteln und Fertigspritzen.

Nach der Herstellung wird die erfindungsgemäß beanspruchte Öl-in-Wasser-Emulsion unter Schutzgas in geeignete Primärpackmittel abgefüllt, um die Produktqualität und - sicherheit zu gewährleisten. Das Abfüllen unter Schutzgas, wie Stickstoff, verhindert die Kontamination durch Luft und minimiert die Oxidation der empfindlichen Inhaltsstoffe, wie beispielsweise der Cannabinoide. Zu den verwendeten Primärpackmitteln gehören Glasvials, Injektionsbeutel, Ampullen und Fertigspritzen. Diese Behälter bieten eine sterile Umgebung und schützen die Emulsion vor äußeren Einflüssen.

Nach dem Abfüllen erfolgt die Sterilisation, um sicherzustellen, dass das Endprodukt frei von Mikroorganismen ist. Häufig verwendete Sterilisationsmethode ist die Autoklavierung. Durch diesen Prozess wird sichergestellt, dass die Öl-in-Wasser-Emulsion den hohen Anforderungen an Sterilität und Sicherheit in der pharmazeutischen Industrie entspricht und für die parenterale Anwendung geeignet ist.

In einer bevorzugten Ausführungsform wir die erfindungsgemäße Öl-in-Wasser-Emulsion durch aseptische Abfüllung mit einem Primärpackmittel verpackt. Bei diesem Verfahren werden die Öl-in-Wasser-Emulsion und die Primärpackmittel unter sterilen Bedingungen zusammengeführt, um eine Kontamination zu vermeiden.

Die erfindungsgemäß beanspruchte Öl-in-Wasser-Emulsion ist mit wenigstens einem der folgenden Vorteile verbunden:
- Die erfindungsgemäß beanspruchte Öl-in-Wasser-Emulsion erlaubt eine definierte Konzentration und kontrollierte Dosierung der Cannabinoide.
- Die erfindungsgemäß beanspruchte Öl-in-Wasser-Emulsion weist eine besonders hohe Stabilität auf, die durch die feine mittlere Teilchengröße von weniger als 500 nm und die kontrollierte Prozessführung erreicht wird.
- Die erfindungsgemäß beanspruchte Öl-in-Wasser-Emulsion erhöht die Bioverfügbarkeit der Cannabinoide durch die feine Verteilung der Öltröpfchen.
- Die erfindungsgemäß beanspruchte Öl-in-Wasser-Emulsion ermöglicht eine kontrollierte Freisetzung der Wirkstoffe, wodurch die Effektivität der Behandlung erhöht und Nebenwirkungen minimiert werden.
- Die erfindungsgemäß beanspruchte Öl-in-Wasser-Emulsion ist aufgrund ihrer hohen Stabilität und kontrollierten Freisetzung einfach in der Handhabung und für verschiedene Verabreichungsschemata geeignet.
- Die erfindungsgemäß beanspruchte Öl-in-Wasser-Emulsion bleibt auch nach 24 Monaten Lagerung bei Raumtemperatur und 60 % relativer Luftfeuchtigkeit stabil, ohne dass es zu einem signifikanten Abbau der Inhaltsstoffe, oder zu einer Phasentrennung kommt.

### BEISPIELE

### Methoden

Bestimmung der mittleren Teilchengröße mittels Photonenkorrelationsspektroskopie (PCS):
Die Photonenkorrelationsspektroskopie (PCS) ist eine Methode zur Charakterisierung von Teilchengrößen in Emulsionen. Bei dieser Technik wird die Brownsche Molekularbewegung der Partikel analysiert, indem die zeitlichen Schwankungen der Lichtstreuung gemessen werden. Ein Laserstrahl wird durch die Probe geleitet, und die Intensität des gestreuten Lichts wird als Funktion der Zeit aufgezeichnet. Diese Intensitätsschwankungen resultieren aus der ungerichteten Diffusion der Teilchen und führen zu zeitabhängigen Interferenzerscheinungen, die von der Größe der Teilchen abhängen. Die Autokorrelationsfunktion des Streulichtsignals ermöglicht die Berechnung der mittleren Tröpfchengröße in Nanometern (nm).

Die Bestimmung des pH-Wertes erfolgt mit einem pH-Meter.

Bestimmung des pFATs Wertes gemäß den Vorgaben der United States Pharmacopeia (USP) <729>.

### Zusammensetzung (Beispiel)

| Komponente | Menge (g) |
|---|---|
| Tetrahydrocannabinol | 100 |
| Sojaöl | 800 |
| Olivenöl | 100 |
| Omega-3-Triglyceride | 100 |
| Mittelkettige Triglyceride | 1000 |
| Ei-Lecithin | 120 |
| Ölsäure | 3 |
| Glycerol | 250 |
| NaOH | 0,4 |
| Wasser | 7500 |

Diese Tabelle zeigt die Modellzusammensetzung einer Öl-in-Wasser-Emulsion mit den angegebenen Komponenten und Mengenangaben.

### Herstellungsverfahren

Bevor die Feststoffe, wie das Cannabinoid und das Ei-Lecithin, in die Ölphase bzw. wässrige Phase eingebracht wurden, mussten diese weitestgehend vom Sauerstoff befreit werden. Dieser Prozess wird als Inertisierung bezeichnet. Hierzu wurde Stickstoff mittels Sonde eingeblasen und wieder abgezogen. Dabei wurde der Sauerstoffgehalt kontinuierlich durch physikalische Gleichgewichtseinstellung gesenkt. Der Sauerstoffgehalt in der Flüssigkeit und/oder im Gasraum wurde mittels Sauerstoffsonden kontrolliert. Der Sauerstoffgehalt war ≤ 3,5 mg/L.

### Herstellung der Wasserphase

Die Wasserphase wurde in einem 7,5-L-Rührkessel hergestellt, der mittels einer Temperierungseinheit über einen Doppelmantel auf 65 °C Prozesstemperatur temperiert wurde. Danach wurden Glycerol und Natriumhydroxid in den Rührkessel dem temperierten Wasser zugegeben und eingerührt.

### Herstellung der Ölphase

Zur Herstellung der Ölphase wurden Sojaöl, mittelkettige Triglyceride (MCT), Olivenöl, und Omega-3-Triglyceride in einem zweiten 5 L Rührkessel gegeben und auf eine Prozesstemperatur von 65 °C temperiert. Danach wurden die Emulgatoren (Ei-Lecithin und Ölsäure) und Tetrahydrocannabinol zugegeben und weiter inertisiert.

### Zusammenführen der Phasen

Vor dem Start der Emulgierung wurden die Prozessparameter für die Dosierung sowie für die Rotor-Stator-Drehzahl an der speicherprogrammierbaren Steuerung (SPS) der Steuerungseinheit des Inline-Rotor-Stator-Dispergators gemäß nachstehender Tabelle eingestellt:

| Prozessparameter | Einstellung |
|---|---|
| Drehzahl | 6000 U/min |
| Dosierung | 40 L/h |
| Gegendruck | 2 bar |
| Temperatur | 65 °C |

Die Ölphase und die Wasserphase wurden dann in den Inline-Rotor-Stator-Dispergator geleitet. Die Vormischung der Ölphase und der Wasserphase zu einer Präemulsion erfolgte im Rotor-Stator-Dispergator. Die mittlere Teilchengröße der Präemulsion lag dabei im Bereich von ≥ 0,5 bis ≤ 500 µm.

### Homogenisierung

Die Präemulsion wurde in einen Hochdruckhomogenisator (Gegenstrahldispergator vom Typ PSI-40) mit drei Durchgängen fein emulgiert. Dieser Hochdruckhomogenisator verwendete eine statische mikrometerdimensionierte Kanalstruktur für den Tropfenaufbruch. Der Tropfenaufbruch fand in einer Interaktionskammer (Scherkammer), bestehend aus einem Diamantkern, statt, der in einer 316L-Edelstahlummantelung versenkt war. Zur Emulsifikation wurden Y-Kammern verwendet. Die Mikrokanäle formten die Form eines Ypsilons, und die Prozessdrücke lagen im Bereich von 500 bar bis 2.000 bar. Zur Vermeidung von Schäden durch Kavitation war der Interaktionskammer ein Auxiliary Processing Module (APM) nachgeschaltet, das als Druckminderer fungierte. Folgende Prozessparameter wurden festgelegt:

| Prozessparameter | Einstellung |
|---|---|
| Druck | 1.000 bar |
| Temperatur | 60 °C |
| Durchgänge | 3 |
| Primärkammer | E101D |
| Sekundärkammer | APM |

Die Kammer E101D war eine Einzelslot-Y-Kammer und lieferte Durchflüsse bis zu 20 L/h. Das APM-Modul lieferte einen Gegendruck von ca. 50 bar für die Primärkammer E101D.

### Abfüllen unter Schutzgas

Die fertige Emulsion wurde unter Schutzgas (z.B. Stickstoff) in Primärpackmittel abgefüllt, um die Produktqualität und -sicherheit zu gewährleisten. Das Abfüllen erfolgte in einem Reinraum bei einer Umgebungstemperatur von etwa 25 °C.

### Sterilisation

Nach dem Abfüllen erfolgte die Sterilisation der Emulsion durch Sterilfiltration bei einem Druck von 3 bar oder Autoklavierung bei 121 °C und 2 bar für 15 Minuten, um sicherzustellen, dass das Endprodukt frei von Mikroorganismen war.

Dieses Verfahren gewährleistete die Herstellung einer stabilen, homogenen und sterilen Öl-in-Wasser-Emulsion, die den hohen Anforderungen der pharmazeutischen Industrie entsprach.

Die Öl-in-Wasser-Emulsion ist auch noch nach 24 Monaten Lagerung bei Raumtemperatur und 60 % relativer Luftfeuchtigkeit stabil.

### Daten

| | |
|---|---|
| pH-Wert | 8,0 |
| PFAT₅ Wert | 0,01 Volumen-% |
| Mittlere Tröpfchengrösse gemäß PCS | 252 nm |
| Säurezahl | ≤ 0,1 KOH/g |
| Peroxidzahl | ≤ 1,0 meq O₂/kg |
| Bakterielle Endotoxine | ≤ 0,5 EU/mL |
| Pyrogene | ≤ 1,15 °C |

Der Anteil Lysophosphatidylcholin war < 500 mg/L. Der Anteil Lysophosphatidylethanolamin war < 500 mg/L.

## Patentansprüche

1. Eine Öl-in-Wasser-Emulsion zur parenteralen Verabreichung umfassend
(a) wenigstens eine Ölphase umfassend wenigstens ein Cannabinoid und wenigstens ein Öl
und
(b) wenigstens eine wässrige Phase,
wobei die Öl-in-Wasser-Emulsion Tröpfchen mit einer mittleren Tröpfchengröße im Bereich von ≥ 50 nm bis ≤ 500 nm, gemessen durch Photonenkorrelationsspektroskopie, aufweist und
einen pFATs Wert ≤ 0,05 Volumen-%, bezogen auf das gesamte Volumen aller Tröpfchen, aufweist.

2. Die Öl-in-Wasser-Emulsion gemäß Anspruch 1, wobei die mittlere Tröpfchengröße im Bereich von ≥ 100 nm bis ≤ 400 nm, bevorzugt im Bereich von ≥ 150 nm bis ≤ 300 nm liegt, gemessen durch Photonenkorrelationsspektroskopie.

3. Die Öl-in-Wasser-Emulsion gemäß Anspruch 1 oder 2, wobei der pFATs Wert ≤ 0,03 Volumen-%, bevorzugt ≤ 0,01 Volumen-% , bezogen auf das gesamte Volumen aller Tröpfchen, beträgt.

4. Die Öl-in-Wasser-Emulsion gemäß wenigstens einem der Ansprüche 1 bis 3, wobei das Öl ausgewählt wird aus der Gruppe bestehend aus pflanzlichen Ölen, Omega-3-Triglyceriden, mittelkettigen Triglyceriden, und deren Kombinationen.

5. Die Öl-in-Wasser-Emulsion gemäß wenigstens einem der vorhergehenden Ansprüche, wobei die Ölphase in einer Menge von ≥ 3,0 bis ≤ 40 Gew.-%, bevorzugt in einer Menge von ≥ 5,0 bis ≤ 30 Gew.-%, besonders in einer Menge von ≥ 10,0 bis ≤ 25 Gew.-%, bezogen auf die gesamte Menge der Öl-in-Wasser-Emulsion, vorliegt.

6. Die Öl-in-Wasser-Emulsion gemäß wenigstens einem der vorhergehenden Ansprüche, wobei das wenigstens eine Cannabinoid ausgewählt wird aus der Gruppe bestehend aus Tetrahydrocannabinol (THC), Δ8-Tetrahydrocannabinol (Δ8-THC), Δ9-Tetrahydrocannabivarin (Δ9-THDV), Cannabidiol (CBD), Cannabinol (CBN), Cannabigerol (CBG), Cannabidivarin (CBDV), Cannabichromen (CBC), Cannabicyclol (CBL), Cannabielsoin (CBE), Cannabitriol (CBT), Cannabinodiol (CBND), Cannabicitran (CBT), Cannabichromanon (CBCN), Tetrahydrocannabinolsäure (THCA), Tetrahydrocannabivarin (THCV) und deren Derivaten.

7. Die Öl-in-Wasser-Emulsion gemäß wenigstens einem der vorhergehenden Ansprüche, wobei das wenigstens eine Cannabinoid in einer Menge von ≥ 0,1 bis ≤ 10,0 Gew.-%, bevorzugt in einer Menge von ≥ 0,5 bis ≤ 8,0 Gew.-%, ganz besonders bevorzugt in einer Menge von ≥ 0,5 bis ≤ 5,0 Gew.-%, noch weiter bevorzug in einer Menge von ≥ 0,5 bis ≤ 2,0 Gew.-%, bezogen auf die gesamte Menge der Öl-in-Wasser-Emulsion, vorliegt.

8. Die Öl-in-Wasser-Emulsion gemäß wenigstens einem der vorhergehenden Ansprüche, wobei der pH-Wert der Öl-in-Wasser-Emulsion im Bereich von ≥ 6,0 bis ≤ 8,5, bevorzugt im Bereich von ≥ 7,5 bis ≤ 8,5, liegt.

9. Die Öl-in-Wasser-Emulsion gemäß wenigstens einem der vorhergehenden Ansprüche, wobei die Säurezahl der Öl-in-Wasser-Emulsion im Bereich von ≥ 0,01 mg KOH/g bis ≤ 2 mg KOH/g liegt.

10. Die Öl-in-Wasser-Emulsion gemäß wenigstens einem der vorhergehenden Ansprüche, wobei die Peroxidzahl der Öl-in-Wasser-Emulsion ≤ 5 meq O₂/kg ist.

11. Die Öl-in-Wasser-Emulsion gemäß wenigstens einem der vorhergehenden Ansprüche, zur Verwendung bei der Behandlung von chronischen Schmerzen, insbesondere bei Krebserkrankungen und -therapie, Multiple Sklerose, Übelkeit, Erbrechen, Appetitanregung und -unterdrückung, Angstzuständen, Schlafstörungen, Fibromyalgie, Gilles-de-la-Tourette-Syndrom, therapieresistentem Glaukom, rheumatoider Arthritis, entzündlichen Darmerkrankungen, insbesondere Morbus Crohn und Colitis ulcerosa, psychotischen Störungen, insbesondere Schizophrenie, Epilepsie und Bewegungsstörungen, Schlaganfall, Parkinson, Migräne, Rückenmarksverletzungen, periphere Neuropathie, und anderen neurogenen Schmerzen, Aufmerksamkeitsdefizit-Syndrom (ADS) und posttraumatischer Belastungsstörung (PTBS).

12. Verfahren zur Herstellung einer Öl-in-Wasser-Emulsion gemäß wenigstens einem der Ansprüche 1 bis 11 umfassend wenigstens die folgenden Schritte:
(A) Inertisieren wenigstens einer Ölphase und wenigstens einer wässrigen Phase, wobei wenigstens ein Cannabinoid der wenigstens einen Ölphase zugesetzt wird,
(B) Erwärmen der wenigstens einen Ölphase auf eine Temperatur im Bereich von ≥ 20 °C bis ≤ 70 °C und erwärmen der wenigstens einen wässrigen Phase auf eine Temperatur im Bereich von ≥ 20 °C bis ≤ 70 °C,
(C) Mischen der wenigstens einen Ölphase und der wenigstens einen wässrigen Phase um eine Präemulsion zu erhalten,
(D) Homogenisieren der Präemulsion, um die Öl-in-Wasser-Emulsion zu erhalten.

13. Das Verfahren gemäß Anspruch 12, wobei Schritt (C) das Mischen der wenigstens einen Ölphase und der wenigstens einen wässrigen Phase unter Verwendung wenigstens eines Rotor-Stator-Dispergators umfasst.

14. Das Verfahren gemäß Anspruch 12 oder 13, wobei Schritt (D) das Homogenisieren der Präemulsion unter Verwendung wenigstens eines Hochdruckhomogenisators umfasst.

15. Das Verfahren gemäß Anspruch 14, wobei der Hochdruckhomogenisator ein Kolben-Spalt-Homogenisator oder ein Gegenstrahldispergator ist.
